# EUROPÄISCHE PATENTSCHRIFT

(11) **EP 2 501 811 B1**
(45) Veröffentlichungstag und Bekanntmachung des Hinweises auf die Patenterteilung: **16.09.2015**
(21) Anmeldenummer: 10777038.0
(22) Anmeldetag: 19.11.2010
(51) Int. Cl.: C12N 15/10

(54) **VERFAHREN ZUR SELEKTIVEN ANREICHERUNG UND ISOLIERUNG MIKROBIELLER UND OPTIONAL ZUSÄTZLICH VIRALER NUKLEINSÄUREN**
Method for selective enrichment and isolation of microbial and optional additionally of viral nucleic acids
Procédé d'enrichissement sélectif et d'isolement de microbes et en outre, optionellement, d'acides nucléiques viraux

(30) Priorität: 19.11.2009 EP 09014446
(43) Veröffentlichungstag der Anmeldung: 26.09.2012
(73) Patentinhaber: QIAGEN GmbH, 40724 Hilden (DE)
(72) Erfinder: HECKEL, Dirk, 40764 Langenfeld (DE); DOEDT, Thomas, 40724 Hilden (DE)
(74) Vertreter: f & e patent
(86) Internationale Anmeldenummer: PCT/EP2010/067787
(87) Internationale Veröffentlichungsnummer: WO 2011/061274

(56) Entgegenhaltungen:
- WO-A1-2009/144182
- DE-A1-102005 009 479
- KELLERMAYER M ET AL: "Release of potassium, lipids, and proteins from nonionic detergent treated chicken red blood cells", JOURNAL OF CELLULAR PHYSIOLOGY, WILEY LISS, NEW YORK, NY, US, Bd. 159, Nr. 2, 1. Mai 1994 (1994-05-01), Seiten 197-204, XP008110703, ISSN: 0021-9541
- "Lexikon der Biochemie - Band 1 A-I", 1999, Spektrum Akademischer Verlag GmbH, Heidelberg * page 179 * * page 456 *

## Beschreibung

Die vorliegende Erfindung betrifft ein Verfahren zur selektiven Anreicherung und/oder Isolierung mikrobieller und optional zusätzlich viraler Nukleinsäuren aus Proben, welche eine Mischung mikrobieller Zellen, höherer eukaryontischer Zellen und frei zirkulierender Nukleinsäuren sowie optional Viren in einer Flüssigkeit enthalten, sowie einen Kit zur selektiven Anreicherung und/oder Isolierung insbesondere intra- sowie extrazellulärer mikrobieller und optional zusätzlich viraler Nukleinsäuren aus Proben, welche eine Mischung mikrobieller und höherer eukaryontischer Zellen, frei zirkulierender Nukleinsäuren, insbesondere extrazellulärer mikrobieller Nukleinsäuren, sowie gegebenenfalls Viren in einer Flüssigkeit enthalten.

Molekularbiologische Analyse- und Diagnoseverfahren gewinnen zunehmend insbesondere in der Human-oder Veterinärmedizin zum Nachweis von Infektionen oder genetischen Krankheitsveranlagungen an Bedeutung, da sie im Gegensatz zu konventionellen Verfahren einen sehr schnellen Nachweis der Erreger und somit eine schnelle Diagnose ermöglichen. Diese ermöglicht wiederum den frühzeitigen Beginn einer gezielten Therapie und kann daher insbesondere bei lebensbedrohlichen Infektionen die Mortalitätsrate der Patienten entscheidend senken. So liegt beispielsweise die Sterblichkeitsrate bei Sepsis-Patienten momentan immer noch bei 20 bis 50%, und Sepsis ist die häufigste Todesursache von in ein Krankenhaus eingewiesenen Menschen. Dieser Umstand ist nicht zuletzt auf die vergleichsweise langwierigen DiagnoseVerfahren zurückzuführen, welche einen Therapie-Beginn unweigerlich verzögern. Traditionell werden zum Nachweis derartiger Infektionen aus den Patienten gewonnene Proben, welche den bzw. die Erreger enthalten, unter geeigneten Bedingungen kultiviert, d.h. auf einem geeigneten Nährmedium angezüchtet, um so eine Vervielfältigung der Mikroorganismen zu erzielen und anschließend die entsprechenden Kolonien nachweisen zu können. Diese Kultivierungsverfahren nehmen im Allgemeinen jedoch mehrere Tage (ca. 3 bis 7 Tage) in Anspruch. Darüber hinaus lassen sich zahlreiche Erreger nicht durch das Anlegen von Blutkulturen nachweisen. Dies gilt insbesondere in der Virusdiagnostik.

Modernere Diagnoseverfahren konzentrieren sich daher auf den spezifischen Nachweis der Erreger-Nukleinsäuren (Desoxyribonukleinsäuren/DNA und/oder Ribonukleinsäuren/RNA). Da mit Hilfe der Polymerase-Kettenreaktion (PCR) binnen weniger Stunden auch sehr geringe Mengen von Nukleinsäuren exponentiell vervielfältigt werden können, ermöglicht dieser Ansatz den Nachweis mikrobieller DNA in humanen Blutproben mit enormer Zeitersparnis, beispielsweise bei der Sepsis-Diagnose, und kann die Überlebenschancen der betroffenen Patienten entscheidend verbessern.

Je nach Art des Erregers befindet sich der größte Teil der nachzuweisenden Nukleinsäuren im Inneren einer Zelle ("intrazelluläre Nukleinsäuren") oder aber frei zirkulierend in der infizierten Probe ("extrazelluläre Nukleinsäuren"). Um die intrazellulären Nukleinsäuren isolieren zu können, müssen diese Nukleinsäuren zunächst aus den Zellen freigesetzt werden, bevor sie aufgereinigt, mit PCR-Techniken vervielfältigt und/oder analysiert werden können. Ein weiteres Problem beim Nachweis mikrobieller DNA aus Proben, welche eine Mischung mikrobieller Zellen und höherer eukaryontischer Zellen enthalten, wie beispielsweise Blutproben von Patienten mit Sepsis, ist ferner die Tatsache, dass bei einer Lyse der Zellen nicht nur die zu analysierenden mikrobiellen Nukleinsäuren aus den in der Probe vorhandenen mikrobiellen Zellen freigesetzt werden, sondern auch große Mengen der eukaryontischen Nukleinsäuren aus den höheren eukaryontischen Zellen, deren Anzahl in einer aus einem höheren Eukaryonten gewonnenen Probe im Allgemeinen ein Vielfaches der Anzahl an mikrobiellen Zellen beträgt. Nukleinsäure-Präparationen, welche aus derartigen Mischproben gewonnen werden, enthalten somit ein Gemisch der Nukleinsäuren aller in der Probe vorhandenen Zelltypen. Das durchschnittliche Verhältnis von mikrobieller zu humaner DNA in Blutproben von menschlichen Sepsis-Patienten beträgt etwa 1 zu 2x10⁹, so dass es in den nachfolgenden Amplifikationsverfahren zum Nachweis der mikrobiellen DNA nicht selten zur Bildung unspezifischer Amplifikationsprodukte kommt, welche eine nachfolgende Diagnostik erschweren oder sogar zu Fehldiagnosen führen können.

Im Stand der Technik gab es bereits verschiedene Ansätze, dieses Problem durch ein geeignetes Verfahren zum Zellaufschluss (Zelllyse) der in einer solchen Probe enthaltenen Zellen zu umgehen. So ist beispielsweise in WO 2006/092278 A1 ein Verfahren beschrieben, welches die selektive Isolierung mikrobieller Nukleinsäuren aus einer Mischprobe ermöglicht, die neben mikrobiellen Zellen höhere eukaryontische Zellen enthält. Die in einer humanen Blutprobe naturgemäß in großer Zahl vorhandenen höheren eukaryontischen Zellen werden hierbei unter Verwendung eines milden Lysepuffers, der ein chaotropes Agenz in relativ niedriger Konzentration enthält, aufgeschlossen, während die ebenfalls in den untersuchten Proben vorhandenen mikrobiellen Zellen zunächst intakt bleiben. Das Verfahren macht sich den Umstand zunutze, dass mikrobielle Zellen im Vergleich zu eukaryontischen Zellen unter chaotropen Bedingungen stabiler sind. Dem erhaltenen Lysat, welches die durch die Lyse freigesetzten eukaryontischen Zellbestandteile sowie die intakten mikrobiellen Zellen enthält, wird anschließend eine geeignete Nuklease zugesetzt, welche die eukaryontische DNA abbaut, wohingegen die intrazelluläre mikrobielle DNA durch die intakte mikrobielle Zelle vor einem Verdau geschützt ist. Durch anschließende Zentrifugation können die mikrobiellen Zellen als Pellet von dem Lysat abgetrennt werden, welches die (zumindest teilweise) abgebaute eukaryontische DNA enthält. In einem nächsten Schritt werden die auf diese Weise erhaltenen mikrobiellen Zellen einem weiteren Aufschluss zur Freisetzung der mikrobiellen intrazellulären DNA unterworfen. Ein Nachteil dieses Verfahrens ist allerdings, dass hierbei virale Nukleinsäuren und die frei zirkulierenden Nukleinsäuren, insbesondere die extrazellulären mikrobiellen Nukleinsäuren, welche sich im flüssigen Bestandteil der Probe befinden, im Falle von Blutproben bspw. im Blutplasma, durch den vorangegangenen Nuklease-Verdau unweigerlich verloren gehen. Dies ist insbesondere im Hinblick darauf von Nachteil, dass neuere Arbeiten gezeigt haben, dass im Plasma befindliche Nukleinsäuren, insbesondere frei zirkulierende Nukleinsäuren, wichtige prognostische Marker darstellen (A. Rhodes et al., Critical Care 2006, 10:R60). Insbesondere bei einer Sepsis weisen die Patienten einen stark erhöhten Gehalt an frei zirkulierenden Nukleinsäuren im Blutplasma auf, der als unabhängiger Indikator zur Vorhersage des Krankheitsverlaufs und der Mortalität des Patienten genutzt werden kann (s. bspw. M. E. Bougnoux et al. J. Clin. Microbiol.1999, 37, 925-930; L. Metwally et al. J. Med. Microbiol. 2008, 57, 1269-1272; K. Saukkonen et al. Clin. Chem. 2008, 54 (6), 1000-1007; Y. K. Tong et al. Clin. Chim. Acta 2006, 363 (1-2), 187-196).

Auch in WO 2009/015484 A1 ist ein Verfahren zur Isolierung von mikrobiellen Nukleinsäuren aus Körperflüssigkeiten beschrieben, welche neben den körpereigenen Zellen Mikroorganismen enthalten. Das dort beschriebene Verfahren umfasst ein zweistufiges Lyseverfahren. In einem ersten Schritt werden unter geeigneten Bedingungen selektiv die Blutzellen lysiert, während die mikrobiellen Zellen intakt bleiben. Anschließend wird die lysierte Probe zentrifugiert, um die intakten mikrobiellen Zellen als Pellet abzutrennen. Nach dem Waschen dieses Pellets können die mikrobiellen Nukleinsäuren aus den in diesem Pellet enthaltenen mikrobiellen Zellen durch eine weitere Lyse freigesetzt werden. Verfahren zur Isolierung mikrobieller Zellen aus biologischen Proben, welche derartige Mischproben aus mikrobiellen und höheren eukaryontischen Zellen enthalten, die auf einem ähnlichen Prinzip beruhen, sind ferner in WO 97/07238 A2, WO 2006/088895 A2 und WO 2008/017097 A1 beschrieben. Keines der dort beschriebenen Verfahren erwähnt jedoch auch nur eine Möglichkeit zur Isolierung der extrazellulären mikrobiellen DNA.

Ein weiterer Ansatz zur Abtrennung intrazellulärer mikrobieller DNA von humaner DNA aus Vollblut-Mischproben liegt dem VYOO-Kit der Firma SIRS-Lab (Jena, Deutschland) zugrunde. Hier wird nach einer mechanischen Lyse der unterschiedlichen in einer Mischprobe enthaltenen Zellen zunächst die Gesamt-DNA der Probe isoliert. Die Anreicherung der mikrobiellen DNA in der Probe erfolgt in einem nächsten Schritt mittels eines festphasengebundenen Antikörpers, welcher die nicht-methylierte DNA der mikrobiellen Erreger bindet, während die methylierte humane DNA nicht gebunden wird. In einem anschließenden Schritt muss die gebundene mikrobielle DNA von der Festphase eluiert werden und anschließend aus dem erhaltenen Eluat ausgefällt werden. Mit diesem Verfahren können zwar sowohl intra- wie auch extrazelluläre mikrobielle Nukleinsäuren angereichert werden, allerdings ist die Anreicherung mit Hilfe der Bindung an die Antikörperfunktionalisierte Festphase nicht quantitativ, und auch beim nachfolgenden Ausfällen gehen signifikante Mengen der mikrobiellen DNA verloren. Ein weiterer Nachteil ist, dass virale Nukleinsäuren auch bei diesem Verfahren nicht aufgereinigt werden. Zudem ist das Verfahren äußerst zeitintensiv.

Aufgabe der vorliegenden Erfindung war es daher, ein Verfahren bereitzustellen zur selektiven Anreicherung und/oder Isolierung mikrobieller Nukleinsäuren aus Proben, welche eine Mischung mikrobieller Zellen und Säugetier- Zellen sowie frei zirkulierender Nukleinsäuren in einer Flüssigkeit enthalten, welches die Anreicherung und/oder Isolierung intra- wie extrazellulärer, insbesondere mikrobieller, Nukleinsäuren aus derselben Probe in kurzer Zeit erlaubt.

Diese Aufgabe wird durch das erfindungsgemäße Verfahren gelöst. Gegenstand der Erfindung ist daher ein Verfahren zur selektiven Anreicherung und/oder Isolierung mikrobieller Nukleinsäuren aus Proben, welche eine Mischung mikrobieller Zellen und Säugetierzellen sowie frei zirkulierender Nukleinsäuren, insbesondere extrazellulärer mikrobieller Nukleinsäuren, in einer Flüssigkeit enthalten, welches die folgenden Schritte umfasst:
Im Wesentlichen Trennen des flüssigen Bestandteils der Probe, einschließlich der in diesem flüssigen Bestandteil gelösten Substanzen wie der frei zirkulierende Nukleinsäuren insbesondere der extrazellulären Nukleinsäuren, von den festen Bestandteilen der Probe, insbesondere von den zellulären Bestandteilen,
   1.
   2. Selektive Lyse der in den festen Probenbestandteilen enthaltenen Säugetierzellen ohne gleichzeitige Lyse der mikrobiellen Zellen, wobei die selektive Lyse der Säugetierzellen in einer Mischung (Puffer 1) durchgeführt wird, umfassend
      1. mindestens eine chaotrope Substanz, ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, Harnstoff oder Mischungen derselben,
      2. mindestens ein nichtionisches Tensid, ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
      3. mindestens eine Puffersubstanz, bevorzugt ausgewählt aus der Gruppe, enthaltend Tris, MES, MOPS, HEPES oder Mischungen derselben,
   in der Weise, dass die Konzentration dieser Substanzen in der Probe nach Zugabe des Puffers (1) 0,1 bis 2 M Chaotrop, (2) 0,5 bis 5 % (w/v) nichtionisches Tensid und (3) 2 bis 50 mM Puffersubstanz beträgt 3.
Im Wesentlichen Trennen der festen Bestandteile, einschließlich der mikrobiellen Zellen, von dem flüssigen Bestandteil, einschließlich der in diesem flüssigen Bestandteil gelösten Substanzen, des in Schritt 2 erhaltenen Lysats,
   4. Resuspension der in Schritt 3 erhaltenen festen Bestandteile,
   5. Lyse der mikrobiellen Zellen, welche in der in Schritt 4 erhaltenen Suspension vorliegen,
   6. Reinigung und/oder Konzentrierung der frei zirkulierenden, insbesondere der extrazellulären mikrobiellen, Nukleinsäuren aus dem in Schritt 1 erhaltenen flüssigen Bestandteil und/oder der intrazellulären mikrobiellen Nukleinsäuren aus dem in Schritt 5 erhaltenen Lysat.

Sofern die Probe neben den mikrobiellen und den Säugetierzellen und den frei zirkulierenden Nukleinsäuren noch Viren enthält, ist ferner optional zusätzlich oder auch gleichzeitig in Schritt 6 eine Anreicherung und/oder Isolierung der viralen Nukleinsäuren möglich, da die Viren hauptsächlich in dem in Schritt 1 des Verfahrens abgetrennten flüssigen Bestandteil der Probe vorliegen sollten_und daher ebenfalls effektiv von den Säugetierzellen abgetrennt werden können.

Besonders bevorzugt werden gemäß dem vorliegenden Verfahren in Schritt 6 sowohl die extra- wie auch die intrazellulären, insbesondere mikrobiellen, Nukleinsäuren aufgereinigt. Dies kann gleichzeitig oder nacheinander separat, oder nach Zusammengeben des flüssigen Bestandteils aus Schritt 1 mit dem Lysat aus Schritt 5 gemeinsam erfolgen.

Mit Hilfe des erfindungsgemäßen Verfahrens können sowohl intrazelluläre als auch extrazelluläre mikrobielle Nukleinsäuren aus Mischproben, welche neben den mikrobiellen Zellen Säugetierzellen und frei zirkulierende Nukleinsäuren enthalten, isoliert und angereichert werden. Hierbei ist möglich, die intrazellulären Nukleinsäuren und/oder die extrazellulären Nukleinsäuren getrennt voneinander oder nach dem Vereinigen der entsprechenden Fraktionen gemeinsam aufzureinigen und zu konzentrieren. Sofern gewünscht, ermöglicht das Verfahren zusätzlich zur selektiven Anreicherung und/oder Isolierung der mikrobiellen Nukleinsäuren ferner auch eine selektive Anreicherung und/oder Isolierung der höheren Säugetier-Nukleinsäuren. Bei den zu isolierenden Nukleinsäuren kann es sich sowohl um RNA als auch um DNA handeln, die jeweils einzelsträngig oder doppelsträngig sein kann. Während für den Nachweis mikrobieller Zellen die Nukleinsäuren bevorzugt DNA darstellen, sind in Abhängigkeit von der Art des Virus (DNA- oder RNA-Virus) für den Nachweis von Viren sowohl DNA als auch RNA von Interesse.

Die mit dem erfindungsgemäßen Verfahren erhaltenen Nukleinsäuren können anschließend durch hoch spezifische und sensitive Verfahren wie PCR- und/oder Hybridisierungsreaktionen nachgewiesen werden. Aufgrund der Abreicherung der humanen gDNA können sehr hohe Eingangsvolumina verwendet werden und die gebundenen Nukleinsäuren gleichzeitig in kleinsten Elutionsvolumina eluiert werden. Dieses hohe Verhältnis von Eingangs- zu Elutionsvolumen führt dann zu einer sehr hohen Sensitivität. Ohne die Abreicherung der humanen gDNA wäre dieses Eingangs-/Elutions-Verhältnis nicht möglich, da es nachfolgend zu PCR-Inhibitionen kommen könnte. Das Verfahren ermöglicht ferner sogar die Freisetzung und den Nachweis phagozytierter mikrobieller Zellen aus Granulozyten oder Makrophagen.

Im Sinne der vorliegenden Erfindung wird unter dem Begriff "selektive Anreicherung und/oder Isolierung" ein Verfahren verstanden, welches es ermöglicht, aus einer Mischprobe, welche mikrobielle Zellen, feri zirkulierende Nukleinsäuren, gegebenenfalls Viren und Säugetierzellen enthält, in reproduzierbarer Weise Nukleinsäuren zur Verfügung zu stellen, welche ausschließlich oder im überwiegenden Maße aus den in der Probe enthaltenen mikrobiellen Zellen stammen, inklusive der extrazellulären mikrobiellen Nukleinsäuren, sowie gegebenenfalls zusätzlich Nukleinsäuren viralen Ursprungs, während der Anteil der Nukleinsäuren aus Säugetierzellen in der Probe gleichzeitig deutlich verringert wird.

Als höhere eukaryontische Zellen werden im Sinne der Erfindung insbesondere Säugetierzellen verstanden, wie sie beispielsweise in tierischen Organismen vorkommen. Derartige multizelluläre Organismen zeichnen sich u.a. durch eine Zelldifferenzierung aus, in dem die einzelne Zelle nicht selbstständig alle lebensnotwendigen biochemischen und metabolischen Funktionen ausführt, sondern durch Spezialisierung an die Ausführung einer oder mehrerer Funktionen angepasst ist. Der Begriff Säugetierzelle umfasst dabei sowohl Zellen, welche in einem Gewebe organisiert sind wie auch einzelne Zellen, beispielsweise Spermatozoen oder Blutkörperchen, die in den Körperflüssigkeiten eines Säugetiers, einschließlich des Menschen, oder in seinen Ausscheidungsprodukten vorkommen, beispielsweise in Blut, Lymphflüssigkeit, Gehirnliquor, in Urin, Faeces oder Speichel. Bevorzugt stellen die Säugetierzellen, humane Zellen dar. Auch Suspensionen oder Aufschlämmungen von Proben in einer wässrigen Lösung, bspw. einem Puffer können als Ausgangsmaterial für das vorliegende Verfahren dienen.

Die zu analysierende Probe enthält eine Mischung mikrobieller Zellen, frei zirkulierender Nukleinsäuren, insbesondere extrazellulärer mikrobieller Nukleinsäuren, gegebenenfalls Viren und Säugetierzellen in einer Flüssigkeit. Vorzugsweise stammt die Probe aus einer natürlichen Umgebung, beispielsweise aus einem pflanzlichen, tierischen oder menschlichen Organismus. Bevorzugt handelt es sich bei der Probe um Körperflüssigkeiten von Säugetieren, besonders bevorzugt von Menschen, umfassend Punktate, Liquor, Lymphflüssigkeit, Gelenk-, Perotoneal- oder Pleuraflüssigkeit, Knochenmark, Blut, insbesondere Vollblut, Sperma, Urin, Faeces, Speichel und Bronchial-Alveolar-Lavage, Konzentrate oder verdünnte Lösungen derselben oder resuspendiertes Zellmaterial, welches durch Abstriche gewonnen wurde. Besonders bevorzugt handelt es sich bei der Probe um Blut oder Blutkonzentrate wie Thrombozytenoder Erythrozytenkonzentrate.

Als mikrobielle Zellen werden im Sinne der Erfindung die diverse Gruppe von Organismen bezeichnet, welche in der Natur selbstständig als einzelne Zelle oder als Zell-Cluster existieren, und sich somit von höheren eukaryontischen Zellen unterscheiden, die in der Natur nicht als einzelne Zelle vorkommen, sondern ausschließlich als Bestandteile multizellulärer Organismen mit Zellspezialisierung. Unter dem Begriff "mikrobielle Zelle" werden im Sinne der Erfindung unter anderem prokaryontische Zellen, beispielsweise Bakterien oder Archaebakterien, aber auch Zellen niederer Eukaryonten wie mikroskopische Algen, Hefen, Pilze oder Protozoen verstanden. In einer bevorzugten Ausführungsform stellen die mikrobiellen Zellen Bakterienzellen und/oder Zellen niederer Pilze dar.

Der Begriff "Bakterien" umfasst hierbei grampositive und gramnegative Bakterien, insbesondere Bakterien der Gattungen *Mycobacterium* spp., *Enterococcus, Enterobacter, Klebsiella, Proteus, Pseudomonas, Serratioa, Escherichia, Acinetobacter, Streptococcus, Staphylococcus, Salmonella, Legionella, Chlamydia, Shigella, Pseudomonas, Listeria, Yersinia, Corynebacterium, Bordetella, Bacillus, Clostridium, Haemophilus, Helicobacter* und *Vibrio.*

Pilzzellen, deren Nukleinsäuren sich mit dem erfindungsgemäßen Verfahren anreichern und oder isolieren lassen, sind insbesondere Zellen von Pilzen der Gattungen *Aspergillus, Basidiobolus, Cephalosporium, Skopulariopsis, Rhizomucor, Entomophthora, Mucor, Syncephalastrum, Absidia, Altenaria, Rhizopus, Stemphylium, Nigrospora, Chrysosporium, Botrytis, Helmithosporium, Curvularia, Hemispora, Phoma, Paecilomyces* und *Thielavia, insbesondere A. fumigatus, A. tereus,* A. *niger, A. nidulans, A. homegadus, A. flavus B. microsporus, B. rhanarum, C. crysogenum, C. corebiobides, C. regatum und C. diospyri.*

Ferner können auch die Nukleinsäuren der pathogenen Hefen der Gattung *Candida,* beispielsweise *C*. *tropicalis*, *C. glabrata, C. krusei, C. parapsilosis, C. albicans, C. guilliermondii* und andere angereichert und/oder isoliert werden, ebenso wie die Nukleinsäuren aus Algen, beispielsweise aus *Euglena* spp., *Coscinodiscus* spp., *Trachelomonas* spp., *Gymnodinium sanguineum, Dinophysis nitra, Ceratium massiliense, Chlorella, Chlorococcum* oder *Eremosphaera,* oder aus Protozoen, wie beispielsweise *Plasmodium, Trypanosoma, Leishmania, Toxoplasma und Cryptosporidium, bevorzugt Plasmodium malariae, Plasmodium falciparum, Trypanosoma brucei, Trypanosoma cruzei, Toxoplasma gondii, Cryptosporidium parvum, Cryptosporidium serpentis* und *Cryptosporidium hominis.*

Des Weiteren können optional auch virale Nukleinsäuren mit dem erfindungsgemäßen Verfahren angereichert und/oder isoliert werden, wie beispielsweise bevorzugt die DNA des Hepatitis-B-Virus (HBV), des Epstein-Barr-Virus (EBV) und des Cytomegalie-Virus (CMV) und/oder die RNA des humanen Immundefizienz-Virus (HIV) und des Hepatitis-C-Virus (HCV).

Das erfindungsgemäße Verfahren ist nicht auf ein spezielles Probenvolumen beschränkt, und es können sowohl Probenvolumina im µl-Bereich wie auch deutlich größere Probenvolumina von beispielsweise 50 oder 100 ml prozessiert werden, wobei die im folgenden genannten Mengen und Volumina der im Verfahren verwendeten Agenzien und Lösungen dann der Probenmenge gegebenenfalls nach dem Fachmann bekannten Maßstäben entsprechend angepasst werden, ebenso wie die Dauer der einzelnen Verfahrensschritte. Bevorzugt beträgt das Volumen der Probe 1 bis 20 ml, weiterhin bevorzugt 2 bis 15 ml und besonders bevorzugt 3 bis 8 ml.

Das Trennen der flüssigen Bestandteile, einschließlich der in diesen flüssigen Bestandteilen gelösten Substanzen, wie beispielsweise frei zirkulierende Nukleinsäuren und gegebenenfalls zusätzlich in der Probe enthaltene Viren, von den festen Bestandteilen in den Verfahrensschritten 1 und 3 des erfindungsgemäßen Verfahrens kann beispielsweise durch Filtration, Zentrifugation oder Dekantieren erfolgen. Hierbei kann das Trennen der flüssigen Bestandteile von den festen Bestandteilen in dem Verfahrensschritt 1 mit der gleichen oder mit einer anderen Methode als im Verfahrensschritt 3 erfolgen. Bevorzugt erfolgt das Trennen der flüssigen Bestandteile von den festen Bestandteilen in den Verfahrensschritten 1 und/oder 3 des erfindungsgemäßen Verfahrens nach den aus dem Stand der Technik bekannten Verfahren zur Gewinnung von Plasma aus Vollblutproben mit Hilfe einer Zentrifugation der Probe. Die Zentrifugation der Probe erfolgt hierbei bevorzugt für 2 bis 30 Minuten, weiterhin bevorzugt für 5 bis 20 Minuten und besonders bevorzugt für 10 bis 15 Minuten. Die Zentrifugation der Probe erfolgt bevorzugt bei einer Beschleunigung von 2.000 bis 10.000 g, bevorzugt bei 5.000 g. Sofern das Trennen der flüssigen Bestandteile von den festen Bestandteilen sowohl im Verfahrensschritt 1 als auch im Verfahrensschritt 3 des erfindungsgemäßen Verfahrens mit Hilfe einer Zentrifugation der Probe erfolgt, können die Beschleunigung und die Dauer der Zentrifugation in den beiden Verfahrensschritten gleich oder verschieden sein. So kann beispielsweise bei der Prozessierung einer Vollblutprobe die Abtrennung des Plasmas, inklusive der in dem Plasma frei zirkulierenden, insbesondere mikrobiellen, Nukleinsäuren und gegebenenfalls der Viren, von den festen Probenbestandteilen, insbesondere den Blutzellen und den mikrobiellen Zellen, in einem ersten Verfahrensschritt durch eine Zentrifugation bei 5.000 g ± 500 g für 10 Minuten erfolgen. Nach Abtrennen des Überstands der zentrifugierten Probe und anschließender selektiver Lyse der in den festen Probenbestandteilen, d.h. im Pellet, enthaltenen Säugetierzellen ohne gleichzeitige Lyse der mikrobiellen Zellen kann der in diesem Schritt erhaltene flüssige Bestandteil (das Lysat), einschließlich der in diesem flüssigen Bestandteil gelösten Substanzen, insbesondere den freigesetzten Zellbestandteilen der Säugetierzellen, von den festen Bestandteilen, einschließlich der mikrobiellen Zellen, durch einen weiteren Zentrifugationsschritt, beispielsweise für 15 Minuten bei 5.000 ± 500 g getrennt werden.

Das Abtrennen des Überstands von den festen Bestandteilen nach der Zentrifugation kann durch jedes beliebige, dem Fachmann zu diesem Zweck bekannte Verfahren erfolgen, beispielsweise durch Dekantieren oder Abpipettieren des Überstands.

Die selektive Lyse der Säugetierzellen in Verfahrensschritt 2 des erfindungsgemäßen Verfahrens wird bevorzugt in einer Mischung (Puffer 1) durchgeführt, welche
1. mindestens eine chaotrope Substanz, ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, Harnstoff oder Mischungen derselben,
2. mindestens ein nichtionisches Tensid, ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
3. mindestens eine Puffersubstanz, bevorzugt ausgewählt aus der Gruppe, enthaltend Tris, MES, MOPS, HEPES oder Mischungen derselben
   umfasst.

Unter dem Begriff "Lyse" wird im Sinne der Erfindung ein Verfahren zum wenigstens teilweisen Aufschluss von Zellen unter zumindest teilweiser Zerstörung der Membranen und/oder Zellwände verstanden, welche die Nukleinsäuren der Zellen umschließen und vom umgebenden Medium trennen. Durch die zumindest teilweise Zerstörung dieser Strukturen werden die Zellbestandteile wie Proteine und Nukleinsäuren in die umgebende Flüssigkeit freigesetzt. Als selektive Lyse der Säugetierzellen wird im Sinne der Erfindung ein Verfahren bezeichnet, bei welchem nur die Säugetierzellen, nicht jedoch die mikrobiellen Zellen in einer Mischprobe lysiert werden, so dass nach erfolgter Lyse nur die intrazellulären Nukleinsäuren der Säugetierzellen in der Mischung vorliegen, während die intrazellulären Nukleinsäuren der mikrobiellen Zellen weiterhin durch die sie umschließende Zellwand geschützt sind. Bevorzugt werden in diesem Schritt der selektiven Lyse mehr als 50 %, weiterhin bevorzugt mehr als 75 %, besonders bevorzugt mehr als 90 % und insbesondere bevorzugt mehr als 98 % der in der Probe befindlichen Säugetierzellen lysiert, während bevorzugt weniger als 50 %, weiterhin bevorzugt weniger als 20 %, besonders bevorzugt weniger als 10 % und insbesondere weniger als 1 % der mikrobiellen Zellen lysiert werden. Derartige selektive Bedingungen lassen sich beispielsweise über die Bestandteile der verwendeten Lysepuffer, die Konzentration der Bestandteile in dem Lysepuffer und die Gesamtkonzentration des Lysepuffers in der Probenlösung einstellen, was dem Fachmann bekannt ist und anhand der später aufgeführten Beispiele verdeutlicht wird.

Als chaotrope Substanz wird eine chemische Verbindung bezeichnet, welche die Fähigkeit hat, die geordneten Wasserstoffbrückenbindungen in Wasser zu stören. Durch die Störung der Wasserstruktur nimmt die Entropie einer wässrigen Lösung zu, und die Löslichkeit unpolarer Substanzen in dieser Lösung wird erhöht. Dadurch werden hydrophobe Wechselwirkungen minimiert, was zu einer Denaturierung von Proteinen und einer Lyse der Zellwände/Plasmamembranen führt. Die chaotropen Substanzen sind beispielsweise der Hoffmeister-Reihe zu entnehmen. Gemäß der Erfindung einzusetzende Substanzen sind die zuvor genannten Guanidiniumverbindungen oder auch Alkaliperchlorate, Alkaliiodide oder Harnstoff. Als Alkaliperchlorate sind im Sinne der Erfindung Natriumperchlorat und Kaliumperchlorat, als Alkaliiodide Natriumiodid oder Kaliumiodid bevorzugt.

Unter einem Tensid wird im Sinne der Erfindung eine organische grenzflächenaktive Substanz verstanden, welche mindestens einen hydrophilen und mindestens einen hydrophoben Teil aufweist. Im Sinne der Erfindung nichtionische Tenside verwendet. Hierbei handelt es sich um ein nichtionisches Tenside oder ein Gemisch verschiedener nichtionischer Tenside, ausgewählt aus Sorbitanester von Fettsäuren wie beispielsweise Sorbitanmonooleat, Sorbitansesquioleat, Sorbitantrioleat, die entsprechenden -laurate, - palmitate, -stearate oder Mischungen derselben, kommerziell bspw. unter den Namen Arlacel 83, Span 65, Span 80 oder Span 85 erhältlich, Polyoxyethylensorbitanester von Fettsäuren, wie bspw. Polyoxyethylensorbitanmonooleat, Polyoxyethylensorbitansesquioleat, Polyoxyethylensorbitantrioleat, die entsprechenden -laurate, -paimitate, -stearate oder Mischungen derselben, kommerziell bspw. unter den Namen Tween 20 oder Tween 85 erhältlich, Polyoxyalkylenether von Fettsäurealkoholen wie bspw. Polyoxyethylenlaurylether, -stearylether, -cetylether, -oleylether oder Mischungen derselben, z.B. Eicosaethylenglycolhexadecylether, kommerziell bspw. unter den Namen Brij 58 oder Brij 98 erhältlich, Polyoxyalkylenether von Alkylphenolen wie bspw. Alkylphenolpoly(glycolether) mit linearen oder verzweigten C₈-Alkylresten, beispielsweise Polyethylenglycol-[4-(1,1,3,3-tetramethylbutyl)phenyl]ether mit 9 bis 10 Ethylenoxid-Einheiten, die kommerziell bspw. unter den Namen Triton X-100, Igepal CA-520 oder Nonidet P40 erhältlich sind, sowie sogenannte Poloxamere, Blockcopolymere aus Ethylenoxid und Propylenoxid, kommerziell bspw. unter dem Namen Pluronic erhältlich. Vorzugsweise kommen in den oben genannten Substanzen unabhängig voneinander mittlere und höhere Fettsäuren bzw. Fettalkohole zur Anwendung, d.h. Fettsäuren bzw. Fettalkohole mit 8-12 bzw. 13-30 C-Atomen. Diese können gesättigt oder ungesättigt sein. Das Tensid erleichtert sowohl die Mischbarkeit der einzelnen Verbindungen innerhalb des Lysepuffers als auch die Mischbarkeit des Lysepuffers mit der Probe. Darüber hinaus kann das Tensid auch aktiv die Zerstörung der Zellwand- oder Plasmamembran-Struktur der Zellen bewirken.

Unter einer Puffersubstanz wird im Sinne der Erfindung ein System aus einer Säure und ihrer konjugierten Base bezeichnet, welche den pH-Wert einer Lösung, enthaltend die Puffersubstanzen, durch Aufnahme bzw. Abgabe von Protonen konstant hält, je nachdem, ob in der Lösung durch eine Reaktion oder externe Zugabe Protonen freigesetzt oder verbraucht werden. Eine Puffersubstanz ist im Sinne der Erfindung somit zu unterscheiden von einer Pufferlösung (auch als Puffer bezeichnet), welche neben dem Lösungsmittel und der eigentlichen Puffersubstanz weitere Substanzen, wie beispielsweise Salze, Tenside und chaotrope Agenzien etc. enthalten kann. Bevorzugt im Sinne der Erfindung sind Puffersubstanzen, welche den pH-Wert der Pufferlösung im Bereich von 6 bis 10, weiter bevorzugt von 7 bis 9, am meisten bevorzugt von 8 stabilisieren. Bevorzugt im Sinne der Erfindung sind weiterhin amphotere Puffersubstanzen, also Verbindungen, welche im selben Molekül sowohl mindestens eine saure wie auch mindestens eine basische Gruppe tragen und somit sowohl sauer als auch basisch reagieren können.

In einer besonders bevorzugten Ausführungsform wird der erfindungsgemäße Puffer 1 als Lysepuffer 1 in der Weise zu der zu lysierenden Probe gegeben, dass die Konzentration der Pufferbestandteile in der Probe nach Zugabe des Puffers (1) 0,1 bis 2 M, vorzugsweise 0,2 bis 1 M, weiter bevorzugt 0,4 bis 0,8 M Chaotrop, bevorzugt ein Gunanidinium-haltiges Chaotrop wie oben beschrieben, (2) 0,5 bis 5 %, vorzugsweise 1 bis 4 %, weiter bevorzugt 1,8 bis 3 % jeweils (w/v) Tensid, bevorzugt ein nicht-ionisches Tensid und (3) 2 bis 50 mM, vorzugsweise 4 bis 30 mM, weiter bevorzugt 10 mM Puffersubstanz, beträgt, am meisten bevorzugt einem pH-Wert von 8. Zum Erreichen derartiger Konzentrationen in der Lyselösung wird ein Puffer eingesetzt, der folgende Konzentrationen aufweist: (1) 0.2 bis 4 M, vorzugsweise 0,4 bis 3 M, weiter bevorzugt 2 M Chaotrop wie oben definiert, vorzugsweise enthaltend Guanidinium, (2) 0.5 bis 10 % , vorzugsweise 1 bis 5 % jeweils (w/v) nichtionisches Tensid wie oben definiert und (3) 2 bis 100 mM, vorzugsweise 10 bis 50 mM Puffersubstanz, vorzugsweise bei einem pH-Wert von 6.0 bis 10.0, weiter bevorzugt von 7.0 bis 9.0 und am meisten bevorzugt von 8.0

Auf die Abtrennung der flüssigen Bestandteile von den festen Bestandteilen in Verfahrensschritt 3 des erfindungsgemäßen Verfahrens, welche bevorzugt durch Zentrifugation erfolgt, ist bereits oben eingegangen worden. Das hierbei erhaltene Pellet, enthaltend die von den flüssigen Bestandteilen abgetrennten festen Bestandteile, vor allem die mikrobiellen Zellen, wird in Schritt 4 des erfindungsgemäßen Verfahrens resuspensiert, beispielweise durch kurzes Schütteln der Probe in dem nach der Abtrennung der flüssigen Bestandteile verbleibenden Restvolumen der Flüssigkeit, um die festen Bestandteile in dieser zu verteilen. Alternativ kann die Probe auch vor oder während des Schüttelns mit einer geringen Menge an Wasser, einer geeigneten Pufferlösung oder einem sonstigen geeigneten Lösungsmittel versehen werden. Außerdem kann ein Zwischenschritt eingeführt werden, in dem das Pellet in einer geeigneten Waschlösung, bspw. in Lysepuffer, gewaschen wird.

Die Lyse der mikrobiellen Zellen in Verfahrensschritt 5 des erfindungsgemäßen Verfahrens kann prinzipiell mit jeder dem Fachmann bekannten Methode erfolgen, welche geeignet ist, die Zellwand und/oder Plasmamembran der mikrobiellen Zellen zumindest teilweise zu zerstören und die in den Zellen enthaltenen Zellbestandteile, insbesondere die Nukleinsäuren, in die umgebende Flüssigkeit freizusetzen. Eine Vielzahl von Verfahren sind für diverse Arten mikrobieller Zellen im Stand der Technik beschrieben, wie beispielsweise das wiederholte Auftauen und Einfrieren, die Behandlung mit Enzymen, wie Lysostaphin, Lyticase oder Lysozym, oder die Zugabe von chemischen Agenzien wie chaotropen Substanzen oder Tensiden in einer entsprechenden Konzentration. Ganz besonders wirksam ist eine Kombination verschiedener Lyseverfahren, die je nach Kompatibilität der Lysemittel gleichzeitig oder sequentiell erfolgen können. Bevorzugt erfolgt die wenigstens teilweise Lyse der mikrobiellen Zellen in Verfahrensschritt 5 des erfindungsgemäßen Verfahrens mechanisch. Geeignete mechanische Verfahren umfassen beispielsweise die Behandlung der mikrobiellen Zellen in einer French-Presse, einer Zellmühle oder einem Mörser sowie eine Ultraschallbehandlung. Besonders bevorzugt erfolgt die Lyse der mikrobiellen Zellen in Verfahrensschritt 5 des erfindungsgemäßen Verfahrens durch mechanisches Homogenisieren der Probe unter Verwendung von geeigneten Partikeln, insbesondere Perlen (beads), bevorzugt Glasperlen. Hierbei ist das mechanische Durchmischen der Probe, enthaltend die mikrobiellen Zellen und die Glasperlen, in einem Gerät bevorzugt, welches diese Probe in horizontaler und/oder vertikaler Ausrichtung schüttelt, wie beispielsweise ein Laborvortexer, ein QIAGEN Tissue-Lyser, ein Fast Prep-Gerät oder ähnliches. Bevorzugt wird das mechanische Homogenisieren der Probe in Gegenwart einer Pufferlösung vorgenommen, die zusätzlich auch lytische Eigenschaften aufweisen kann. In einer besonders bevorzugten Ausführungsform erfolgt die Lyse der mikrobiellen Zellen in Verfahrensschritt 5 des erfindungsgemäßen Verfahrens unter Verwendung von Glasperlen einer Größe von 400 bis 600 µm in Gegenwart einer Pufferlösung, insbesondere eines Puffers mit lytischen Eigenschaften.

Eine geeignete Pufferlösung zur Verwendung bei der oben beschriebenen mechanischen Lyse, die zusätzlich lytische Eigenschaften aufweist (Puffer 2) umfasst hierbei bevorzugt
1. mindestens eine Puffersubstanz, bevorzugt ausgewählt aus der Gruppe enthaltend Tris-HCl, MES, MOPS, HEPES oder Mischungen derselben,
2. mindestens ein Komplexierungsreagenz für zweiwertige Metallionen, bevorzugt EDTA,
3. mindestens ein Tensid, bevorzugt ein nichtionisches Tensid, besonders bevorzugt ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
4. optional einen Schauminhibitor, bevorzugt ausgewählt aus der Gruppe, enthaltend Reagenz DX (QIAGEN, Hilden, Deutschland) und SILFOAM® SRE (Wacker Chemie, München, Deutschland) oder ähnliche geeignete Schauminhibitoren,
und weist einen pH-Wert von 6 bis 10, vorzugsweise von 7 bis 9, am meisten bevorzugt von 8 auf.

Die Bedeutung der Begriffe Puffersubstanz und Tensid sowie bevorzugte Ausführungsformen im Sinne der vorliegenden Erfindung wurden bereits oben für den Lysepuffer 1 erläutert. Die für den Puffer 1 genannten Ausführungsformen sind ebenso als Bestandteile des Puffers 2 bevorzugt, wobei als nichtionisches Tensid für den Puffer 2 besonders bevorzugt Polyoxyalkylenether von Alkylphenolen wie bspw. Alkylphenolpoly(glycolether) mit linearen oder verzweigten C₈-Alkylresten zum Einsatz kommt, kommerziell erhältlich unter dem Namen Triton X-100.

Weiterhin enthält der Puffer 2 ein Komplexierungsreagenz, insbesondere für zweiwertige Metallionen. Hierfür sind alle Substanzen geeignet, die in der Lage sind, Metallionen und insbesondere zweiwertige Metallionen, zu komplexieren, beispielsweise DTPA, EGTA oder EDTA, wobei letzteres bevorzugt ist.

Optional kann der Puffer 2 weiterhin einen Schauminhibitor enthalten, um ein zu starkes Schäumen der Probe während der mechanischen Lyse zu verhindern, welches im Extremfall zum ungewollten Austritt der Probe aus dem Probengefäß und damit zu Substanzverlust führen würde und weiterhin auch die nachfolgende Prozessierung der Probe u. a. aufgrund der Volumenzunahme erschweren würde. Bevorzugt im Sinne der Erfindung sind beispielsweise die kommerziell erhältlichen Schauminhibitoren Reagenz DX (QIAGEN, Hilden, Deutschland) und SILFOAM® SRE (Wacker Chemie, München, Deutschland). Sofern der Lysepuffer einen Schauminhibitor enthält, ist dieser bevorzugt in einem Anteil von 0.1 bis 1 % (v/v), bezogen auf die Gesamtzusammensetzung des Puffers, enthalten.

Besonders bevorzugt umfasst der Puffer 2, der vorteilhafterweise auch lytische Eigenschaften aufweist" (1) eine Puffersubstanz, (2) Komplexierungsreagenz, (3) Tensid und (4) optional einen Schauminhibitor wie Reagenz DX und weist einen pH-Wert von 7.0 bis 9.0 auf. Es ist bevorzugt, dass die jeweiligen Pufferkomponenten während der mechanischen Lyse in einer Konzentration von (1) 2 bis 20 mM, vorzugsweise 4 bis 15 mM, weiter bevorzugt 5 bis 10 mM Puffersubstanz, (2) 0,1 bis 5 mM, vorzugsweise 1 bis 4 mM, weiter bevorzugt 2 bis 3 mM Komplexierungsreagenz, (3) 0,1 bis 5 %, vorzugsweise 1 bis 3 %, weiter bevorzugt 1,8 bis 2 % jeweils (w/v) Tensid, vorzugsweise nichtionisches Tensid, und (4) optional 0,1 bis 1 %, vorzugsweise 0,3 bis 0,9 %, weiter bevorzugt 0,5 bis 0,7 % jeweils (v/v) Schauminhibitor vorzugsweise bei einem pH-Wert von 7.0 bis 9.0, weiter bevorzugt von 8.0 vorliegen. Eine geeignete Pufferlösung, die derartige Lysebedingungen liefert, enthält vorzugsweise (1) 5 bis 50 mM, vorzugsweise 8 bis 25 mM, weiter bevorzugt 10 bis 20 mM Puffersubstanz, (2) 0.5 bis 10 mM, vorzugsweise 1 bis 8 mM, weiter bevorzugt 2 bis 5 mM Komplexierungsmittel, (3) 0.2 bis 10 %, vorzugsweise 0,5 bis 5 %, weiter bevorzugt 1,2 bis 2 % jeweils (w/v) Tensid und (4) optional 0.1 bis 3 %, vorzugsweise 0,2 bis 2 %, weiter bevorzugt 0,5 bis 1 % jeweils (v/v) Schauminhibitor wie beispielsweise Reagenz DX vorzugsweise bei einem pH-Wert von 7.0 bis 9.0, weiter bevorzugt bei 8.0.

Da es in der Praxis vorkommen kann, dass das obige Lyseverfahren nicht zu einer vollständigen, sondern nur zu einer teilweisen Lyse der mikrobiellen Zellen führt, ist daher in einer bevorzugten Ausführungsform ein weiteres Lyseverfahren nachgeschaltet, das vorzugsweise mit einer Lyselösung erfolgt. Es ist jedoch auch möglich die Lyse der mikkrobiellen Zellen direkt mit der entsprechenden Lyselösung zu bewirken, ohne vorab eine mechanische Lyse durchzuführen.

Da es besonders vorteilhaft ist, im Rahmen der Reinigung und Konzentrierung insbesondere der mikrobiellen und ggf. viralen Nukleinsäuren, einen Protease-Verdau durchzuführen, kann dieser in günstiger Weise mit dem beschriebenen Lyseschritt kombiniert werden. Es ist jedoch auch denkbar, den Protease-Verdau in einem anderen Verfahrensschritt, beispielsweise in Kombination mit der mechanischen Lyse, oder im anschließenden Reingungs- und/oder Konzentrierungsprozess durchzuführen.

Vorteilhafterweise wird zur Optimierung der Lyse der mikrobiellen Zellen entsprechend Verfahrensschritt 5 die Lyse zumindest teilweise durch Versetzen der Suspension aus Verfahrensschritt 4 oder durch Versetzen des Lysates resultierend aus der oben beschriebenen vorherigen mechanischen Lyse, die gegebenenfalls unterstützt wird durch einen ebenfalls zumindest teilweise lytisch wirkenden Puffer, mit einer Protease, bevorzugt Proteinase K, und einer Lyselösung (Puffer 3) bewirkt. Vorzugsweise umfasst Puffer 3
1. mindestens eine chaotrope Substanz, bevorzugt ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, HArnstoff oder Mischungen derselben,
2. mindestens ein Tensid, bevorzugt ein nichtionisches Tensid, besonders bevorzugt ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
3. mindestens eine Puffersubstanz, bevorzugt Tris, MES, MOPS, HEPES oder Mischungen derselben.

Die dabei erhaltene Mischung wird zur Aktivierung der Protease vorzugsweise für 5 bis 40 Minuten bei 45° bis 70°C, weiter bevorzugt für 10 bis 20 Minuten bei 50 bis 65°C, besonders bevorzugt für 15 Minuten bei 60°C inkubiert. Bevorzugt wird die Probe hierzu mit einem Volumen des Puffers 3 versetzt, welches 40 bis 150 %, bevorzugt 70 bis 110 % und insbesondere 90 % ihres eigenen Volumens beträgt, sowie mit einem Volumen einer Proteaselösung, bevorzugt einer Proteinase K - Lösung mit > 600 mAU/ml (AU: Anson unit), kommerziell beispielsweise als QIAGEN Proteinase K (QIAGEN, Hilden, Deutschland) erhältlich, welches 1 bis 30 %, bevorzugt 5 bis 20 % und besonders bevorzugt 10 % ihres eigenen Volumens beträgt. So werden beispielsweise zu 5 ml der Probe in einer besonders bevorzugten Ausführungsform 4 ml des Puffers 3 und 0,5 ml Proteaselösung hinzugegeben.

Besonders gute Ergebnisse werden erzielt, wenn die oben genannten Substanzen der Pufferlösung während der Lyse insbesondere in einer Konzentration von (1) 1 bis 5 M Chaotrop, (2) 5 bis 20 % (w/v) Tensid und (3) 20 bis 100 mM Puffersubstanz, insbesondere bei einem pH-Wert von 8.0, vorliegen.

Ein geeigneter Puffer, der derartige Lysekonditionen liefert, enthält (1) 3 bis 10 M, vorzugsweise 4 bis 8 M, weiter bevorzugt 4,5 bis 6 M Chaotrop, (2) 10 bis 40 %, vorzugsweise 15 bis 30 %, weiter bevorzugt 20 bis 25 % jeweils (w/v) Tensid und (3) 40 bis 200 mM, vorzugsweise 50 bis 150 mM, weiter bevorzugt 70 bis 100 mM Puffersubstanz, insbesondere bei einem pH-Wert von 8.0

In einer besonders bevorzugten Ausführungsform wurde der beschriebene Puffer 3 bereits dem flüssigen Bestandteil aus Verfahrensschritt 1 zugegeben.

In einer bevorzugten Ausführungsform des erfindungsgemäßen Verfahrens werden sowohl die intrazellulären mikrobiellen als auch die frei zirkulierenden, insbesondere die extrazellulären mikrobiellen, Nukleinsäuren und besonders bevorzugt zusätzlich virale Nukleinsäuren angereichert und/oder isoliert.

Hierzu kann der in dem Verfahrensschritt 1 des erfindungsgemäßen Verfahrens abgetrennte flüssige Bestandteil, enthaltend die frei zirkulierenden, insbesondere die extrazellulären mikrobiellen, Nukleinsäuren und gegebenenfalls die viralen Nukleinsäuren, mit dem in Verfahrensschritt 5 erhaltenen Lysat, enthaltend die intrazellulären mikrobiellen Nukleinsäuren, vor der Reinigung und/oder Konzentrierung der Nukleinsäuren gemäß Verfahrensschritt 6 des erfindungsgemäßen Verfahrens vereinigt werden. In diesem Fall kann der in dem Verfahrensschritt 1 des erfindungsgemäßen Verfahrens abgetrennte flüssige Bestandteil, enthaltend die frei zirkulierenden, insbesondere extrazellulären mikrobiellen, Nukleinsäuren und gegebenenfalls die viralen Nukleinsäuren, bevorzugt bereits unmittelbar nach seiner Abtrennung in den oben beschriebenen Puffer 3 gegeben und darin aufbewahrt werden. Das wie oben beschrieben in einem optional vorgelagerten mechanischen Lyseschritt erhaltene Lysat, enthaltend die intrazellulären mikrobiellen Nukleinsäuren, kann dann anschließend in zur Optimierung der Lyse zu diesem Gemisch gegeben werden.

Alternativ können der in Verfahrensschritt 1 des erfindungsgemäßen Verfahrens erhaltene flüssige Bestandteil, enthaltend die frei zirkulierenden, insbesondere extrazellulären mikrobiellen, Nukleinsäuren und gegebenenfalls die viralen Nukleinsäuren und das in Verfahrensschritt 5 erhaltene Lysat, enthaltend die intrazellulären mikrobiellen Nukleinsäuren, voneinander getrennt gereinigt und/oder konzentriert werden. Sofern der in Verfahrensschritt 1 erhaltene flüssige Bestandteil auch Viren enthält und eine Isolierung der viralen Nukleinsäuren gewünscht ist, wird dieser flüssige Bestandteil bevorzugt noch einem zusätzlichen Schritt zur Freisetzung der viralen Nukleinsäuren unterzogen. Verfahren hierfür sind dem Fachmann bekannt und umfassen typischerweise eine chemische Lyse und/oder einen Proteinase-Verdau.

Die Protease für den Protease-Verdau, vorzugsweise die Proteinase K, insbesondere in Form eines Puffers, kann entweder sowohl der Suspension aus Verfahrensschritt 4 bzw. dem Lysat resultierend aus der oben beschriebenen vorherigen mechanischen Lyse als auch dem in Verfahrensschritt 1 erhaltenen flüssigen Bestandteil in jeweils getrennten Probenansätzen zugegeben werden. Alternativ können das Lysat der mechanischen Lyse und der flüssige Bestandteil aus Verfahrensschritt 1, optional in Kombination mit Puffer 3, vor Zugabe des Protease-haltigen Puffers vereint werden.

Es ist ebenfalls möglich, den Protease-haltigen Puffer auch nur dem Lysat der mechanischen Lyse zuzugeben. Die extrazellulären Nukleinsäuren aus dem flüssigen Bestandteil aus Verfahrensschritt 1 können dann anschließend mit den im Lysat der nachgeschalteten chemischen Lyse befindlichen intrazellulären Nukleinsäuren gemeinsam (vereint) oder getrennt voneinander gemäß der nachfolgend beschriebenen weiteren Schritte 6a - 6d aufgereinigt und/oder konzentriert werden.

Die Reinigung und/oder Konzentrierung der mikrobiellen und viralen Nukleinsäuren gemäß Schritt 6 des erfindungsgemäßen Verfahrens umfasst bevorzugt folgende Schritte:
6a) Versetzen des Lysates aus Verfahrensschritt 5 mit einer Mischung (Bindepuffer), bevorzugt umfassend eine Mischung aus (1) einem linearen oder verzweigten C₁- bis C₄-Alkohol, insbesondere Methanol, Ethanol, Propanol, Isopropanol oder n- Butanol, iso-Butanol, sek. Butanol oder tert. Butanol, bevorzugt Isopropanol, und (2) einem Puffer entsprechend Puffer 3, bevorzugt in einem Verhältnis von 30 - 80 Vol.% des linearen oder verzweigten C₁- bis C₄-Alkohols zu 20 - 70 Vol.% Puffer 3 und Inkubation des resultierenden Gemisches;
6b) Aufbringen der Probe auf eine geeignete chromatographische Vorrichtung, bevorzugt auf eine chromatographische Vorrichtung zur selektiven Bindung der mikrobiellen und gegebenenfalls viralen Nukleinsäuren durch Adsorption, besonders bevorzugt auf Silicabasis;
6c) optional einmaliges oder mehrmaliges Waschen der gebundenen mikrobiellen und gegebenenfalls viralen Nukleinsäuren, bevorzugt mit einer ersten Lösung (Waschpuffer 1), welche (1) mindestens eine chaotrope Substanz, bevorzugt ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide oder Mischungen derselben, (2) einen linearen oder verzweigten C₁-C₄-Alkohol, bevorzugt Ethanol, und (3) mindestens ein chaotropes Salz, bevorzugt in einer Konzentration von 2 bis 4 M, umfasst, vorzugsweise gefolgt von einer zweiten Lösung (Waschpuffer 2), welche (1) einen linearen oder verzweigten C₁-C₄-Alkohol, bevorzugt Ethanol, (2) ein nicht chaotropes Salz, bevorzugt Natriumchlorid, in einer Konzentration von 10 bis 200 mM und (3) 1 bis 25 mM einer Puffersubstanz, bevorzugt Tris, MES, MOPS, HEPES oder Mischungen derselben (pH 7.5) umfasst, aber keine chaotrope Substanz enthält;
6d) optional Elution der mikrobiellen und gegebenenfalls viralen Nukleinsäuren vorzugsweise mit Wasser oder einer Lösung (Elutionspuffer), umfassend (1) mindestens eine Puffersubstanz und (2) mindestens ein Komplexierungsreagenz für Metallionen, insbesondere für zweiwertige Metallionen. Ebenso können andere herkömmliche Elutionsmittel Verwendung finden.

Das Volumen des zu der Probe hinzugegebenen Bindepuffers beträgt bevorzugt 100 bis 500 % des Probenvolumens, bevorzugt 200 bis 400 % des Probenvolumens und besonders bevorzugt 320 % des Probenvolumens. Die anschließende Inkubation des resultierenden Gemischs erfolgt bevorzugt für 1 bis 20 Minuten bei -5 bis 5°C, besonders bevorzugt für 2 bis 10 Minuten bei 0°C und insbesondere für 5 Minuten bei 0°C.

Die Abtrennung der erhaltenen mikrobiellen und gegebenenfalls viralen Nukleinsäuren von den restlichen Probenbestandteilen erfolgt bevorzugt durch die selektive Anbindung der mikrobiellen und gegebenenfalls viralen Nukleinsäuren an eine feste Matrix durch Adsorption, bevorzugt durch Adsorption an eine Matrix auf Silica-Basis, welche bei Zusatz eines geeigneten Bindepuffers im wesentlichen selektiv die in einer Lösung befindlichen Nukleinsäure-Moleküle bindet, während Proteine und weitere in der Lösung befindliche Zellbestandteile und Pufferbestandteile wie Salze, Puffersubstanzen, Tenside etc. in Lösung verbleiben und durch die Waschlösung von den gebundenen Nukleinsäuren abgetrennt werden können. Unter den Begriffen "selektive Bindung" und "selektive Anbindung" von Nukleinsäuren ist im Sinne der Erfindung durch eine Matrix ist im Sinne der Erfindung die spezifische Wechselwirkung (chemische oder physikalische Bindung durch Komplexierung, Adhäsion etc.) von Nukleinsäuremolekülen mit der Matrix zu verstehen, während die Matrix nicht oder nur in geringerem Maße mit weiteren Probenbestandteilen, die keine Nukleinsäuren darstellen, bspw. Salze, Proteine etc., wechselwirkt. Es ist hingegen nicht erforderlich, dass sich die Wechselwirkung der Matrix mit Nukleinsäuren unterschiedlichen Ursprungs unterscheidet, also ein Unterschied zwischen der Art und Stärke der Wechselwirkung bei mikrobiellen Nukleinsäuren im Vergleich zu eukaryontischen Nukleinsäuren vorliegen müsste. Geeignete Systeme sind dem Fachmann bekannt und in einer Vielzahl von Ausgestaltungen kommerziell erhältlich, beispielsweise auch in Ausführungen, die selektiv nur die in einer Probe vorliegende DNA binden, während die in der Probe vorliegenden RNA-Moleküle wie die Proteine und die weiteren in der Probe vorhandenen Proben- und Pufferbestandteile in Lösung verbleiben. Derartige Ausführungen sind besonders bevorzugt, wenn lediglich DNA, jedoch keine RNA isoliert werden soll. Besonders bevorzugt im Sinne der Erfindung sind beispielsweise QIAamp DNA Minisäulen oder die im QIAamp Circulating Nucleic Acid Kit enthaltenen QIAamp Minisäulen (Qiagen, Hilden, Deutschland), welche bevorzugt in Form einer sog. Spin-Säule ausgestaltet sind, bei der das Waschen und die Elution auf einfache und schnelle Weise durch Zentrifugation oder Anlegen von Vakuum erfolgen kann. Das zum optionalen Waschen der gebundenen mikrobiellen und gegebenenfalls viralen Nukleinsäuren verwendete Volumen der Waschpuffer 1 und 2 ist abhängig von der Größe und Dimension der in Schritt 6b verwendeten chromatographischen Vorrichtung sowie der aufgetragenen Probenmenge. Entsprechende Angaben können den Protokollen des jeweiligen Herstellers entnommen werden. Im Falle einer QIAamp DNA Mini Säule beträgt das bevorzugte Volumen der Waschlösungen 1 und 2 beispielsweise 500 bis 900 µl zur Reinigung einer Probe, welche aus einem ursprünglichen Probenvolumen von 10 ml humanen Vollblutes gewonnen wurde.

Bevorzugt wird die Säule nach dem Waschen der gebundenen Nukleinsäuren getrocknet, bevor die Nukleinsäuren in Schritt 6d) des erfindungsgemäßen Verfahrens optinal von der Säule eluiert werden können. Hierfür kann die Säule beispielsweise mit 500 bis 900 µl Ethanol gespült werden, anschließend bei maximaler Geschwindigkeit zentrifugiert und dann bei Raumtemperatur oder erhöhter Temperatur (40 bis 65 °C) getrocknet werden.

Der in Schritt 6d) des erfindungsgemäßen Verfahrens optional verwendete Elutionspuffer umfasst bevorzugt Tris-HCl und EDTA, besonders bevorzugt 2 bis 20 mM Tris-HCl und 0.1 bis 1 mM EDTA, insbesondere 10 mM Tris-HCl und 0.5 mM EDTA. Auch das Volumen des Elutionspuffers hängt sowohl von der Art und Dimension der chromatographischen Vorrichtung, als auch von der auf die chromatographische Vorrichtung auftragenen Probenmenge ab. Bevorzugte Werte können beispielsweise den Angaben des Herstellers der chromatographischen Vorrichtung entnommen werden. Bei Verwendung einer QIAamp DNA Mini Säule und einer ursprünglich eingesetzten Probenmenge von etwa 10 ml Vollblut beträgt das Volumen des Elutionspuffers bevorzugt 20 bis 200 µl. Bevorzugt wird die Säule nach Zugabe des Elutionspuffers kurz bei Raumtemperatur (ca. 1 bis 5 Minuten) inkubiert, bevor die Elution der Nukleinsäuren durch Zentrifugieren bei maximaler Geschwindigkeit, bspw. 0.5 bis 3 Minuten bei 20.000 g, erfolgt.

Generell ist es jedoch auch möglich, die gebundene Nukleinsäure ohne vorherige Elution weiter zu prozessieren und/oder zu analysieren.

Die vorliegende Erfindung stellt weiterhin einen Kit zur selektiven Anreicherung und/oder Isolierung intrazellulärer mikrobieller sowie frei zirkulierender, inssbesondere extrazellulärer mikrobieller, Nukleinsäuren sowie gegebenenfalls zusätzlicher viraler Nukleinsäuren aus Proben bereit, welche eine Mischung mikrobieller und Säugetierzellen und frei zirkulierender Nukleinsäuren, insbesondere extrazellulärer mikrobieller Nukleinsäuren, sowie gegebenenfalls zusätzlich Viren in einer Flüssigkeit enthalten, umfassend:
1. einen ersten Puffer vorzugsweise mit lytischen Eigenschaften (Puffer 1), welcher bereits oben näher beschrieben wurde,
2. mindestens einen zweiten Puffer (Puffer 2), vorzugsweise mit lytischen Eigenschaften, und/oder einen weiteren Puffer (Puffer 3), vorzugsweise mit lytischen Eigenschaften, welche ebenfalls bereits oben näher beschrieben wurden, und
3. Glasperlen, bevorzugt von 400 bis 600 µm Durchmesser, besonders bevorzugt von 500 µm Durchmesser.

In einer bevorzugten Ausführungsform umfasst der Kit weiterhin eine chromatographische Vorrichtung zur Reinigung und/oder Konzentrierung der mikrobiellen und gegebenenfalls viralen Nukleinsäuren, bevorzugt eine chromatographische Vorrichtung zur selektiven Bindung von DNA und/oder RNA durch Adsorption, besonders bevorzugt auf Silica-Basis, sowie weiterhin einen Bindepuffer, bevorzugt den bereits definierten Bindepuffer, optional einen oder mehrere Waschpuffer, bevorzugt die beiden bereits oben definierten Waschpuffer und optional einen Elutionspuffer, bevorzugt den bereits oben definierten Elutionspuffer. Optional kann der Kit weiterhin Komponenten zum Nachweis der angereicherten und/oder isolierten Nukleinsäuren in einem sogenannten Multiplex-Verfahren (Multiplex-Assay) enthalten, bspw. für einen Nachweis der erhaltenen DNA in einer Multiplex-PCR mehrere Primer-Paare, eine geeignete DNA-Polymerase, Desoxyribonucleosidtriphosphate, eine Quelle für Mg²⁺-Ionen und/oder für die PCR geeignete Pufferlösungen.

### Beschreibung der Abbildungen

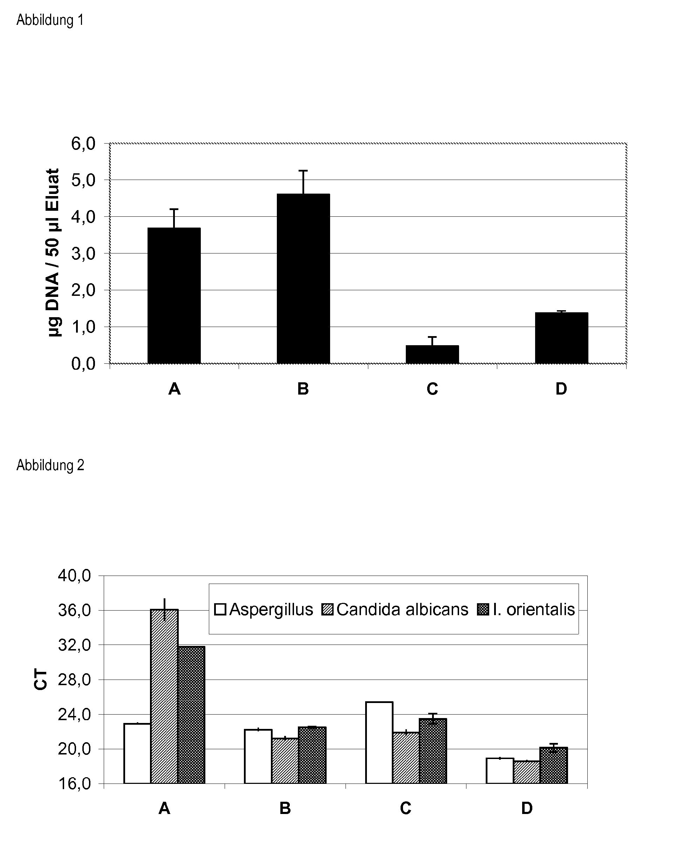

Abbildung 1 zeigt die erhaltene Gesamtmenge DNA (d. h. die Summe humaner und Pilz-DNA, wobei die humane DNA den Hauptanteil ausmacht), welche aus mit isolierter Pilz-DNA und Pilzzellen (freier *Aspergillus* DNA sowie Zellen von *C*. *albicans* und *I. orientalis)* versetzten humanen Vollblutproben in vier unterschiedlichen Verfahren A bis D (s. Beispiel 1) gewonnen wurde. Die der Probe A zugrunde liegende Probemenge betrug 500 µl Vollblut, für Probe B wurde 1 ml Vollblut verwendet. Für die Proben C und D wurden von einer Probenmenge von 5 bzw. 10 ml Vollblut ausgegangen, wobei bei Probe C nur die nach Abtrennen der flüssigen Bestandteile (des Blutplasmas) verbleibenden festen Probenbestandteile unter Abreicherung humaner Nukleinsäuren prozessiert und gereinigt wurden, während in Probe D vor der chromatographischen Reinigung die lysierten mikrobiellen Zellen mit dem im Schritt 1 des erfindungsgemäßen Verfahrens abgetrennten flüssigen Bestandteil der Probe, enthaltend die extrazellulären mikrobiellen Nukleinsäuren, vereinigt wurden. Während die Proben A und B mittels herkömmlicher Verfahren ohne Abreicherung der humanen gDNA behandelt wurden und daher einen hohen Gehalt an humaner gDNA aufweisen, ist der Gehalt an humaner gDNA in den Proben C und D deutlich geringer, obwohl das Probenvolumen der Proben C und D das 5- bis 20-fache des Probenvolumens der Proben A und B betrug.

Abbildung 2 zeigt das Ergebnis der Vervielfältigung der mikrobiellen DNA der in Abbildung 1 gezeigten Proben A bis D mittels Multiplex-Echtzeit-PCR (Multiplex-RT-PCR) anhand der erhaltenen CT-Werte (s. Beispiel 1).

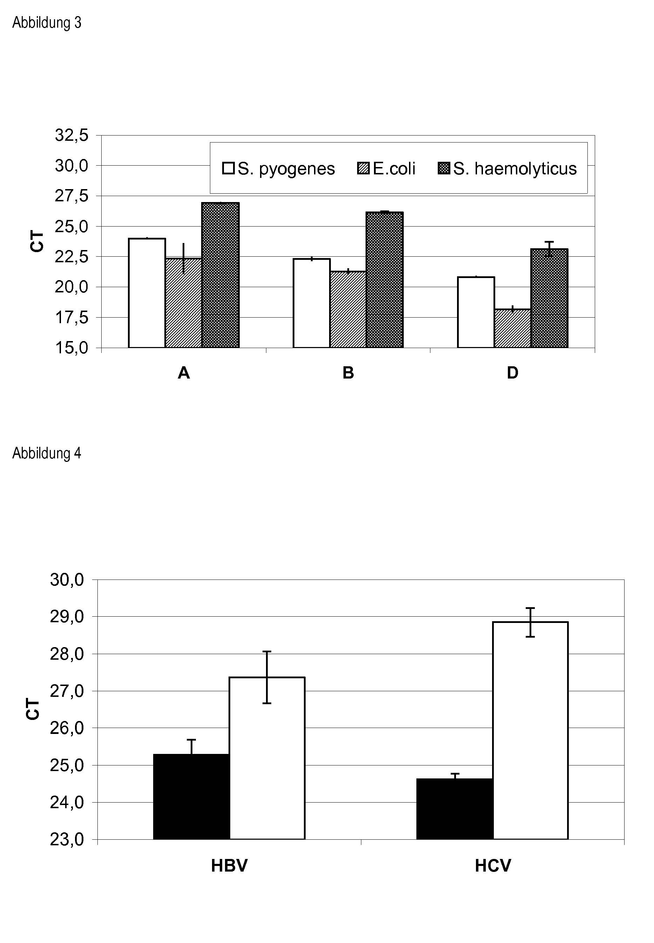

Abbildung 3 zeigt das Ergebnis der Vervielfältigung bakterieller DNA mittels Multiplex-RT-PCR, welche aus mit isolierter bakterieller DNA und Bakterienzellen (freier S. *haemolyticus* DNA sowie Zellen von *E.coli* und *S. pyogenes*) versetzten humanen Vollblutproben in drei unterschiedlichen Verfahren A, B und D (s. Beispiel 2) gewonnen wurde. Die der Probe A zugrunde liegende Probemenge betrug 500 µl Vollblut, für Probe B wurde 1 ml Vollblut verwendet. Für die Probe D wurde von einer Probenmenge von 10 ml Vollblut ausgegangen, wobei vor der chromatographischen Reinigung die lysierten mikrobiellen Zellen mit dem im Schritt 1 des erfindungsgemäßen Verfahrens abgetrennten flüssigen Bestandteil der Probe, enthaltend die extrazellulären mikrobiellen Nukleinsäuren, vereinigt wurden. Der CT-Wert der erfindungsgemäß behandelten Probe D ist auch bei der Isolierung bakterieller DNA deutlich geringer.

Abbildung 4 zeigt das Ergebnis der Vervielfältigung von mit Hilfe des erfindungsgemäßen Verfahrens erhaltenen viralen Nukleinsäuren (als schwarze Balken dargestellt) mittels Multiplex-RT-PCR bzw. Multiplex-RT-Reverse-Transkriptase PCR im Vergleich Nukleinsäuren, die unter Verwendung eines kommerziell erhältlichen Kits zum Nachweis viraler Nukleinsäuren gewonnen wurde (weiße Balken) für Desoxyribonukleinsäuren anhand von Hepatitis B-Viren (HBV) und Ribonukleinsäuren anhand von Hepatitis C-Viren (HCV) (s. Beispiel 3).

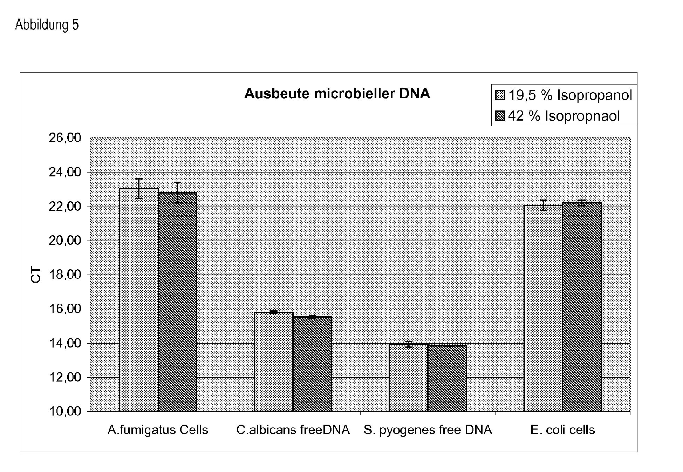

Abbildung 5 zeigt das Ergebnis der Vervielfältigung der mikrobiellen DNA der in Beispiel 4 aufgearbeiteten Proben mittels Multiplex-Echtzeit-PCR (Multiplex-RT-PCR) anhand der erhaltenen CT-Werte (s. Beispiel 4). Die ersten beiden Balken zeigen die erhaltenen CT-Werte der isolierten DNA aus A. fumigatus ganzen Zellen, die zweiten beiden Balken die CT-Werte der isolierten DNA aus C. albicans (freie DNA), die dritten beiden Balken CT-Werte der isolierten DNA aus S.pyrogenes (freie DNA) und die rechten beiden Balken die CT-Werte der isolierten DNA aus E. coli ganzen Zellen. Die Balken stellen jeweils links das Ergebnis bei 19,5% Isopropanol und rechts das Ergebnis bei 42% Isopropanol dar.

### Beispiele:

### Beispiel 1: Selektive Anreicherung und Isolierung von Pilz-DNA aus Vollblutproben

Vollblut wurde mit freier *Aspergillus* DNA (1 x 10⁴ Genomäquivalente/ml Vollblut) und Zellen von *C*. *albicans* und *I. orientalis* (jeweils 1 x 10⁴ Zellen/ml Vollblut) versetzt. Zur Isolierung der Pilz-DNA wurden Teilmengen des versetzten Blutes auf insgesamt vier verschiedene Arten A bis D aufbereitet:
A (Vergleichsverfahren ohne Abreicherung der humanen Nukleinsäuren und ohne mechanischen Prälyseschritt): 500 µl des versetzten Vollblutes wurden mit 250 µl eines Lysepuffers 2, enthaltend 20 mM Tris-HCl, 2 mM EDTA, 1.2 % Triton X-100 und 0.5 % Reagenz DX, versetzt und durchmischt. Die erhaltene Mischung wurde mit 500 µl des kommerziell erhältlichen Lysepuffers AL (QIAGEN, Hilden, Deutschland) sowie 50 µl Qiagen Proteinase K versetzt und 10 min bei 56 °C inkubiert. Anschließend wurden die Nukleinsäuren unter Verwendung des QIAamp Blood Mini Kits (QIAGEN, Hilden, Deutschland) nach Herstelleranweisung unter Verwendung der in dem Kit enthaltenen Minisäulen und Wasch- und Elutionspuffer isoliert, wobei die an die Säulen gebundenen Nukleinsäuren mit je 750 ml Puffer AW1 und Puffer AW2 gewaschen und mit 50 µl Puffer AE eluiert wurden.
B (Vergleichsverfahren ohne Abreicherung der humanen Nukleinsäuren): 1 ml des versetzten Vollblutes wurden mit 500 µl des Lysepuffers 2 sowie 800 mg Glasbeads eines Durchmessers von 500 µm versetzt und 10 min durch Vortexen durchmischt und dabei mechanisch aufgeschlossen. 500 µl des erhaltenen Gemisches wurden mit 500 µl des kommerziell erhältlichen Lysepuffers AL (QIAGEN, Hilden, Deutschland) sowie 50 µl Qiagen Proteinase K versetzt und 10 min bei 56 °C inkubiert. Anschließend wurden die Nukleinsäuren unter Verwendung des QIAamp Blood Mini Kits (QIAGEN, Hilden, Deutschland) nach Herstelleranweisung unter Verwendung der in dem Kit enthaltenen Minisäulen und Wasch- und Elutionspuffer isoliert, wobei die an die Säulen gebundenen Nukleinsäuren mit je 750 ml Puffer AW1 und Puffer AW2 gewaschen und mit 50 µl Puffer AE eluiert wurden.
C: 5 ml des versetzten Vollbluts wurden mit 1.9 ml eines Lysepuffers 1 versetzt, enthaltend 1.5 M GTC, 6.7 % Brij 58 und 33 mM Tris (pH 8), und das Gemisch wurde 10 min bei Raumtemperatur inkubiert und anschließend 10 min bei 5000 Upm zentrifugiert. Der nach dem Zentrifugieren erhaltene Überstand wurde bis auf etwa 50 µl verworfen. Das verbleibende Pellet wurde in etwa 450 µl Puffer PBS (enthaltend 137 mM Natriumchlorid, 2,7 mM Kaliumchlorid und 12 mM Gesamt-Phosphat aus Dinatriumhydrogenphosphat und Kaliumdihydrogenphosphat) resuspendiert. Das erhaltene Gemisch (ca. 500 µl) wurde in ein neues Gefäß, enthaltend 250 µl des Lysepuffers 2 und 400 mg Glassbeads eines Durchmessers von etwa 425 - 600 µm transferiert. Die Probe wurde 10 min durch Vortexen gemischt und dabei mechanisch aufgeschlossen. 500 µl des erhaltenen Überstandes wurden mit 500 µl des kommerziell erhältlichen Lysepuffers AL (QIAGEN, Hilden, Deutschland) sowie 50 µl Qiagen Proteinase K versetzt und 10 min bei 56 °C inkubiert. Anschließend wurden die Nukleinsäuren unter Verwendung des QIAamp Blood Mini Kits (QIAGEN, Hilden, Deutschland) nach Herstelleranweisung unter Verwendung der in dem Kit enthaltenen Minisäulen, Wasch- und Elutionspuffer isoliert, wobei die an die Säulen gebundenen Nukleinsäuren mit je 750 ml Puffer AW1 und Puffer AW2 gewaschen und nach dem Trocknen der Säule mit 50 µl Puffer AE eluiert wurden.
D (erfindungsgemäßes Verfahren):
   10 ml des versetzten Vollbluts wurden zum Abtrennen des Blutplasmas, einschließlich der in dem Blutplasma befindlichen extrazellulären Nukleinsäuren, 10 Minuten bei etwa 5.000 g zentrifugiert. Nach erfolgter Zentrifugation wurde die obere Plasmaphase (ca. 4.5 ml) abgetrennt und in ein neues Gefäß überführt. Das Volumen der festen Bestandteile wurde durch Zugabe des Puffer PBS auf 10 ml aufgefüllt. Nach Zugabe von 3.75 ml Puffer des Lysepuffers 1, wurde das erhaltene Gemisch 10 min bei Raumtemperatur inkubiert. Der milde Lysepuffer lysiert lediglich die humanen Zellen, während die mikrobiellen Zellen intakt bleiben. Anschließend wurde die Probe 15 min bei etwa 5.000 Upm zentrifugiert. Der erhaltene Überstand wurde bis auf 250 µl abgenommen und verworfen. Dieser Überstand enthält v. a. humane DNA und kann alternativ auch für eine separate differentielle Nukleinsäure-Aufreinigung weiter verwendet werden. Das verbleibene Pellet wurde mit jeweils 250 µl des Puffers PBS und des Lysepuffers 2 versetzt. 400 mg Glasbeads einer Größe von 425 - 600 µm wurden hinzugegeben, und die Mischung wurde 10 min durch Vortexen oder bei 50 Hz mit dem QIAGEN TissueLyser LT durchmischt und dabei mechanisch aufgeschlossen. 500 µl des Überstandes wurden zu der zuvor abgetrennten Plasmafraktion gegeben (möglich ist es auch, das mechanisch aufgeschlossene Proben-Glasperlen-Gemisch über einen groben Filter direkt in die Plasmafraktion zu filtrieren, um den Verlust an flüssigem Probenmaterial möglichst gering zu halten) und mit 4 ml Puffer eines Lysepuffers 3, enthaltend 4.5 M GTC, 20 %Brij 58 und 100 mM Tris (pH 8), und 500 µl QIAGEN Proteinase K versetzt. Die erhaltene Mischung wurde 20 min bei 60 °C inkubiert, mit 9 ml eines Bindepuffers, enthaltend 1.2 M GTC, 5,5 % Brij 58, 27.6 mM Tris und 72 % (v/v) Isopropanol, versetzt, durch Vortexen durchmischt und 5 min auf Eis inkubiert. Die Probe wurde anschließend unter Vakuum mit Hilfe eines Aufsatzes, der das Aufbringen von Probenvolumina auf eine Säule erlaubt, die größer als das Volumen der Probe selbst sind, bspw. ein 20 ml Extender (QIAgen, Hilden, Deutschland) auf eine QIAamp Mini Säule aufgezogen, wobei die in der Probe vorhandenen Nukleinsäuren selektiv an die Säule gebunden wurden. Die an die Säulen gebundenen Nukleinsäuren wurden dann mit je 750 ml der kommerziell erhältlichen Puffer AW1 und Puffer AW2 (QIAGEN, Hilden, Deutschland) gewaschen, die Säule wurde getrocknet, und die Nukleinsäuren wurden mit 50 µl des kommerziell erhältlichen Puffers AE (QIAGEN, Hilden, Deutschland) eluiert. Die Gesamtmenge an DNA (humane und Pilz-DNA) in dem Eluat wurde UV-Vis-spektroskopisch bestimmt (Abbildung 1). Zum Nachweis der Pilz-DNA wurden jeder gereinigten Probe 8 µl entnommen und einer Multiplex-PCR unterworfen. Die Ergebnisse sind in Abbildung 2 dargestellt.

### Beispiel 2: Selektive Anreicherung und Isolierung bakterieller DNA aus Vollblutproben

Vollblut wurde mit freier S. *haemolyticus* DNA (1 x 10⁴ Genomäquivalente/ml Vollblut) und den Zellen von E. *coli* und S. *pyogenes* (jeweils 1 x 10⁴ Zellen/ml Vollblut) versetzt. Zur Isolierung der bakteriellen DNA wurden Teilmengen des versetzten Blutes auf insgesamt drei verschiedene Arten A, B und D wie in Beispiel 1 beschrieben aufbereitet. Zum Nachweis der bakteriellen DNA wurden jeder gereinigten Probe 7.5 µl entnommen und einer Multiplex-PCR unterworfen. Die Ergebnisse sind in Abbildung 3 dargestellt.

### Beispiel 3 - Selektive Anreicherung und Isolierung viraler Nukleinsäuren aus Vollblutproben

Humanes Vollblut wurde mit kommerziell erhältlichen HBV- und HCV-Standards (Acrometrix, Benicia, Kalifornien, USA) versetzt, so dass jeweils eine Viren-Konzentration von 5,0 x 10⁴ IU/ml (International Units pro Milliliter) in der Blutprobe erhalten wurde.

Zur Isolierung der viralen Nukleinsäuren wurden die Proben auf zwei verschiedene Arten aufbereitet: Zum einen wurden 500 µl Blutplasma, welches durch Zentrifugieren eines Teils des versetzten Blutes erhalten wurde, mittels des kommerziell erhältlichen QIAGEN DSP Virus Kits (QIAGEN, Hilden, Deutschland) nach Herstellerangaben aufbereitet. Des Weiteren wurden 8 ml des versetzten Vollbluts nach dem erfindungsgemäßen Verfahren aufbereitet, wie unter D in Beispiel 1 beschrieben. Der Nachweis und die Quantifizierung der erhaltenen viralen Nukleinsäuren wurden mittels RT-PCR und Echtzeit-Reverse-Transkriptase-PCR vorgenommen. Die Ergebnisse sind in Abbildung 4 dargestellt.

### Beispiel 4 - Isolierung mikrobieller Nukleinsäuren mit unterschiedlicher Isopropanol-Konzentration im Bindepuffer

Humanes Vollblut wurde mit entweder mit freier DNA von *Aspergillus fumigatus* und *Candida albicans* oder mit lebenden Zellen von S. *pyogenes* und *E.coli* versetzt. Zunächst wurde die gDNA aus den menschlichen Blutzellen aus diesen Proben gemäß dem oben unter D beschriebenen erfindungsgemäßen Verfahren abgereichert und die mikrobiellen Zellen in den Proben, die diese enthielten, wie oben beschrieben aufgeschlossen. Zu den kombinierten Plasmafraktionen (je 5 ml gesamt) wurden jeweils 4 ml Lysepuffer 3 und 0,5 ml Proteinase K-Lösung zugegeben. Die erhaltene Mischung wurde 20 min bei 60 °C inkubiert, mit 9 ml eines Bindepuffers, enthaltend 1.2 M GTC, 5,5 % Brij 58, 27.6 mM Tris und Isopropanol, versetzt, durch Vortexen durchmischt und 5 min auf Eis inkubiert, wobei der Bindepuffer entweder eine Isopropanol-Konzentration aufwies, die eine Isopropanol-Konzentration von ca. 19,5 % oder von ca. 42% Isopropanol im Bindeansatz bewirkte. (Der Bindepuffer enthielt jeweils zunächst 5,4 ml des Lysepuffers 1.2 M GTC, 5,5 % Brij 58, 27.6 mM Tris und 3,6 ml Isopropanol. Für den Ansatz mit erhöhter Isopropanol-Konzentration wurden in den Ansatz zusätzliche 7 ml Isopropanol zugegeben). Die weitere Aufarbeitung der Proben erfolgte wie oben beschrieben.

Zum Nachweis und zur Quantifizierung der erhaltenen microbiellen Nukleinsäuren wurden jeder gereinigten Probe 7.5 µl entnommen und einer Multiplex-PCR unterworfen. Die Ergebnisse sind Tabelle 1 und Abbildung 5 zu entnehmen. Die Durchflusszeiten der Proben durch die Silica-Säulchen betrugen 10,5 min bei 19,5% Isopropanol bzw. 6,5 min bei 42% Isopropanol.

**Tabelle 1: CT-Mittelwerte**

| Probe | *A. fumigatus* Zellen | *C. albicans* Freie DNA | *S. pyrogenes* Freie DNA | *E. coli* Zellen |
|---|---|---|---|---|
| 19,5 % Isopropanol | 23,04 ±0,57 | 15,81±0,07 | 13,93±0,16 | 22,06±0,29 |
| 42 % Isopropnaol | 22,80±0,60 | 15,53±0,08 | 13,84±0,02 | 22,20±0,16 |

## Patentansprüche

1. Verfahren zur selektiven Anreicherung und/oder Isolierung mikrobieller Nukleinsäuren aus Proben, welche eine Mischung mikrobieller Zellen, Säugertierzellen und frei zirkulierender Nukleinsäuren, insbesondere extrazellulärer mikrobieller Nukleinsäuren, in einer Flüssigkeit enthalten, umfassend die folgenden Schritte:
1. Im Wesentlichen Trennen des flüssigen Bestandteils der Probe, einschließlich der in diesem flüssigen Bestandteil gelösten Substanzen wie der frei zirkulierende Nukleinsäuren insbesondere der extrazellulären mikrobiellen Nukleinsäuren, von den festen Bestandteilen der Probe, insbesondere von den zellulären Bestandteilen,
2. Selektive Lyse der in den festen Probenbestandteilen enthaltenen Säugetierzellen ohne gleichzeitige Lyse der mikrobiellen Zellen, wobei die selektive Lyse der Säugetierzellen in einer Mischung (Puffer 1) durchgeführt wird, umfassend
1. mindestens eine chaotrope Substanz, ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, Harnstoff oder Mischungen derselben,
2. mindestens ein nichtionisches Tensid, ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
3. mindestens eine Puffersubstanz, bevorzugt ausgewählt aus der Gruppe, enthaltend Tris, MES, MOPS, HEPES oder Mischungen derselben,
in der Weise, dass die Konzentration dieser Substanzen in der Probe nach Zugabe des Puffers (1) 0,1 bis 2 M Chaotrop, (2) 0,5 bis 5 % (w/v) nichtionisches Tensid und (3) 2 bis 50 mM Puffersubstanz beträgt.
3. Im Wesentlichen Trennen der festen Bestandteile, einschließlich der mikrobiellen Zellen, von dem flüssigen Bestandteil, einschließlich der in diesem flüssigen Bestandteil gelösten Substanzen, des in Schritt 2 erhaltenen Lysats,
4. Resuspension der in Schritt 3 erhaltenen festen Bestandteile,
5. Lyse der mikrobiellen Zellen, welche in der in Schritt 4 erhaltenen Suspension vorliegen,
6. Reinigung und/oder Konzentrierung der frei zirkulierenden, insbesondere der extrazellulären mikrobiellen Nukleinsäuren aus dem in Schritt 1 erhaltenen flüssigen Bestandteil und/oder der intrazellulären mikrobiellen Nukleinsäuren aus dem in Schritt 5 erhaltenen Lysat.

2. Verfahren gemäß Anspruch 1, wobei die Säugetierzellen humane Zellen darstellen.

3. Verfahren gemäß Anspruch 1 oder 2, wobei die Proben Körperflüssigkeiten, Konzentrate oder verdünnte Lösungen derselben oder resuspendiertes Zellmaterial, welches durch Abstriche gewonnen wurde, darstellen, bevorzugt Blut oder Blutkonzentrate.

4. Verfahren gemäß irgendeinem der Ansprüche 1 bis 3, wobei die mikrobiellen Zellen Bakterien- und/oder Pilzzellen darstellen.

5. Verfahren gemäß irgendeinem der Ansprüche 1 bis 4, wobei das Im Wesentlichen Trennen der flüssigen Bestandteile von den festen Bestandteilen in den Verfahrensschritten 1 und/oder 3 gemäß Anspruch 1 mit Hilfe von Zentrifugation, Filtration und/oder Dekantieren der Probe erfolgt.

6. Verfahren gemäß irgendeinem der Ansprüche 1 bis 5, wobei die selektive Lyse der Säugetierzellen gemäß Verfahrensschritt 2 des Anspruchs 1 bei einem pH-Wert von 8.0 durchgeführt wird.

7. Verfahren nach irgendeinem der Ansprüche 1 bis 6, wobei die Lyse der mikrobiellen Zellen in Verfahrensschritt 5 gemäß Anspruch 1 zumindest teilweise mechanisch erfolgt, bevorzugt durch mechanisches Homogenisieren der Probe unter Verwendung von Partikeln, vorzugsweise Glasperlen, besonders bevorzugt unter Verwendung von Glasperlen einer Größe von 400 bis 600 µm in Gegenwart einer Pufferlösung.

8. Verfahren gemäß Anspruch 7, wobei die Pufferlösung (Puffer 2)
1. mindestens eine Puffersubstanz, bevorzugt ausgewählt aus der Gruppe enthaltend Tris-HCl, MES, MOPS, HEPES oder Mischungen derselben,
2. mindestens ein Komplexierungsreagenz für Metallionen, insbesondere für zweiwertige Metallionen, bevorzugt EDTA,
3. mindestens ein Tensid, bevorzugt ein nichtionisches Tensid, besonders bevorzugt ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
4. optional einen Schauminhibitor, bevorzugt ausgewählt aus der Gruppe enthaltend Reagenz DX und SILFOAM SRE,
umfasst und einen pH-Wert von 6 bis 10, bevorzugt 7.0 bis 9.0 aufweist,
und wobei die Substanzen der Pufferlösung während der mechanischen Lyse insbesondere in einer Konzentration von (1) 2 bis 20 mM Puffersubstanz, (2) 0,1 bis 5 mM Komplexierungsreagenz, (3) 0,1 bis 5 % (w/v) Tensid und (4) optional 0,1 bis 1 % (v/v) Schauminhibitor bei einem pH-Wert von 8.0 vorliegen.

9. Verfahren nach irgendeinem der Ansprüche 1 bis 8, wobei die Lyse der mikrobiellen Zellen in Verfahrensschritt 5 gemäß Anspruch 1 zumindest teilweise erfolgt durch Versetzen der Suspension aus Verfahrensschritt 4. gemäß Anspruch 1 oder des Lysates resultierend aus Anspruch 7 oder 8 mit einer Proteinase, bevorzugt Proteinase K, und einer Pufferlösung (Puffer 3), umfassend
1. mindestens eine chaotrope Substanz, bevorzugt ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, Harnstoff oder Mischungen derselben,
2. mindestens ein Tensid, bevorzugt ein nichtionisches Tensid, besonders bevorzugt ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und
3. mindestens eine Puffersubstanz, bevorzugt ausgewählt aus der Gruppe enthaltend Tris, MES, MOPS, HEPES oder Mischungen derselben,
4. und gegebenenfalls den flüssigen Bestandteil aus Verfahrensschritt 1 gemäß Anspruch 1, und wobei die Substanzen der Pufferlösung während der Lyse insbesondere in einer Konzentration von (1) 1 bis 5 M Chaotrop, (2) 5 bis 20 % (w/v) Tensid und (3) 20 bis 100 mM Puffersubstanz, insbesondere bei einem pH-Wert von 8.0, vorliegen
und Inkubation des erhaltenen Gemisches.

10. Verfahren gemäß irgendeinem der Ansprüche 1 bis 9, zusätzlich umfassend die Anreicherung und/oder Isolierung viraler Nukleinsäuren aus Proben, welche neben einer Mischung mikrobieller Zellen, Säugetierzellen und frei zirkulierender Nukleinsäuren, insbesondere extrazellulärer mikrobieller Nukleinsäuren, in einer Flüssigkeit zusätzlich Viren enthalten, wobei die Viren in Verfahrensschritt 1 gemäß Anspruch 1 gemeinsam mit dem flüssigen Bestandteil der Probe von den festen Probenbestandteilen abgetrennt werden.

11. Verfahren gemäß einem der Ansprüche 1 bis 10, wobei der in Verfahrensschritt 1 gemäß Anspruch 1 abgetrennte flüssige Bestandteil, enthaltend die extrazellulären, bevorzugt mikrobiellen, Nukleinsäuren und gegebenenfalls die viralen Nukleinsäuren, mit dem in Verfahrensschritt 5 gemäß Anspruch 1 erhaltenen Lysat, enthaltend die intrazellulären mikrobiellen Nukleinsäuren, vor der Reinigung und/oder Konzentrierung der Nukleinsäuren gemäß Verfahrensschritt 6 des Anspruchs 1 vereinigt wird.

12. Verfahren gemäß einem der Ansprüche 1 bis 10, wobei der in Verfahrensschritt 1 gemäß Anspruch 1 erhaltene flüssige Bestandteil, enthaltend die extrazellulären mikrobiellen und gegebenenfalls die viralen Nukleinsäuren und das in Verfahrensschritt 5 gemäß Anspruch 1 erhaltene Lysat, enthaltend die intrazellulären mikrobiellen Nukleinsäuren, voneinander getrennt gereinigt und/oder konzentriert werden.

13. Verfahren gemäß irgendeinem der Ansprüche 1 bis 12, wobei die Reinigung und Konzentrierung der mikrobiellen und gegebenenfalls viralen Nukleinsäuren gemäß Schritt 6 des Anspruchs 1 folgende Schritte umfasst:
6a) Versetzen des Lysates aus Verfahrensschritt 5 gemäß Anspruch 1 und gegebenenfalls des flüssigen Bestandteils aus Schritt 1 mit einer Lösung (Bindepuffer), bevorzugt umfassend eine Mischung aus (1) einem linearen oder verzeigten C₁- bis C₄-Alkohol, bevorzugt Isopropanol, und (2) einem Puffer 3 gemäß Anspruch 9, enthaltend (1) 3 bis 6 M Chaotrop, (2) 10 bis 30 % (w/v) Tensid und (3) 50 bis 150 mM Puffersubstanz, insbesondere bei einem pH-Wert von 8.0, bevorzugt in einem Verhältnis von 30 - 80% linearem oder verzeigtem C₁- bis C₄-Alkohol zu 20 - 70% Puffer 3 und Inkubation des resultierenden Gemisches,
6b) Aufbringen der Probe auf eine geeignete chromatographische Vorrichtung, bevorzugt eine chromatographische Vorrichtung zur selektiven Bindung der mikrobiellen und gegebenenfalls viralen Nukleinsäuren durch Adsorption, besonders bevorzugt auf Silkabasis,
6c) optional einmaliges oder mehrmaliges Waschen der gebundenen mikrobiellen und gegebenenfalls viralen Nukleinsäuren, ,
6d) optional Elution der mikrobiellen und gegebenenfalls viralen Nukleinsäuren.

14. Ein Kit zur selektiven Anreicherung und/oder Isolierung intrazellulärer mikrobieller sowie frei zirkulierender, insbesondere extrazellulärer mikrobieller, Nukleinsäuren und gegebenenfalls zusätzlich viraler Nukleinsäuren aus Proben, welche eine Mischung mikrobieller Zellen, Säugetierzellen und frei zirkulierender Nukleinsäuren, insbesondere extrazellulärer mikrobieller Nukleinsäuren, sowie gegebenenfalls zusätzlich Viren in einer Flüssigkeit enthalten, umfassend:
1. einen ersten Puffer gemäß Anspruch 1, enthaltend (1) 0.2 bis 4 M Chaotrop, ausgewählt aus der Gruppe, enthaltend Guanidiniumthiocyanat (GTC), Guanidiniumhydrochlorid (GuHCl), Guanidiniumisothiocyanat (GIT), Alkaliperchlorate, Alkaliiodide, Harnstoff oder Mischungen derselben, (2) 0.5 bis 10 % (w/v) nichtionisches Tensid, ausgewählt aus der Gruppe, enthaltend Sorbitanester von Fettsäuren, Polyoxyethylensorbitanester von Fettsäuren, Polyoxyalkylenether von Fettsäurealkoholen, Polyoxyalkylenether von Alkylphenolen, Poloxamere oder Mischungen derselben und (3) 2 bis 100 mM Puffersubstanz, vorzugsweise mit einem pH-Wert von 8.0,
2. mindestens einen zweiten Puffer gemäß Anspruch 8, enthaltend (1) 5 bis 50 mM Puffersubstanz, (2) 0.5 bis 10 mM Komplexierungsreagenz, (3) 0.2 bis 10 % (w/v) Tensid und (4) optional 0.1 bis 3 % (v/v) Schauminhibitor bei einem pH-Wert von 8.0 , und/oder gemäß Anspruch 9, enthaltend (1) 3 bis10 M Chaotrop, (2) 10 bis 40 % (w/v) Tensid und (3) 40 bis 200 mM Puffersubstanz, insbesondere bei einem pH-Wert von 8.0,
3. Glasperlen, bevorzugt von 400 bis 600 µm Durchmesser, besonders bevorzugt von 500 µm Durchmesser.

15. Kit gemäß Anspruch 14, weiterhin umfassend eine chromatographische Vorrichtung zur Reinigung und/oder Konzentrierung der mikrobiellen und gegebenenfalls viralen Nukleinsäuren, bevorzugt eine chromatographische Vorrichtung zur selektiven Bindung der mikrobiellen und viralen Nukleinsäuren durch Adsorption, besonders bevorzugt auf Silikabasis, sowie weiterhin einen Bindepuffer, bevorzugt einen Bindepuffer gemäß Anspruch 13, optional einen oder mehrere Waschpuffer, optional einen Elutionspuffer, sowie optional weitere Komponenten zum Nachweis der angereicherten und/oder isolierten Nukleinsäuren.

## Claims

1. Method for the selective enrichment and/or isolation of microbial nucleic acids from samples that contain a mixture of microbial cells, mammalian cells and freely circulating nucleic acids, in particular extracellular microbial nucleic acids, in a liquid, comprising the following steps:
1. Essentially separating the liquid components from the sample, including the substances dissolved in this liquid component such as the freely circulating nucleic acids, in particular the extracellular microbial nucleic acids, from the solid components of the sample, in particular from the cellular components,
2. Selective lysis of the mammalian cells contained in the solid components of the sample without, at the same time, lysis of the microbial cells, wherein the selective lysis of the mammalian cells is carried out in a mixture (buffer 1), comprising
1. at least one chaotropic substance selected from the group containing guanidine thiocyanate (GTC), guanidine hydrochloride (GuHCl), guanidine isothiocyanate (GIT), alkali perchlorates, alkali iodides, urea or mixtures thereof,
2. at least one non-ionic surfactant, selected from the group containing sorbitan esters of fatty acids, polyoxyethylene sorbitan esters of fatty acids, polyoxyalkylene ethers of fatty acid alcohols, polyoxyalkylene ethers of alkyl phenols, poloxamers or mixtures thereof, and
3. at least one buffer substance, preferably selected from the group containing Tris, MES, MOPS, HEPES or mixtures thereof,
in such a way that the concentration of these substances in the sample, after adding the buffer, consist of (1) 0.1 to 2 M of chaotrope, (2) 0.5 to 5 % (w/v) of non-ionic surfactant and (3) 2 to 50 mM of buffer substance,
3. Essentially separating the solid components, including the microbial cells, from the liquid component, including the substances dissolved in this liquid component, of the lysate obtained in step 2,
4. Resuspension of the solid components obtained in step 3,
5. Lysis of the microbial cells which are present in the suspension obtained in step 4,
6. Purification and/or concentration of the freely circulating, in particular extracellular, microbial nucleic acids from the liquid component obtained in step 1 and/or the intracellular microbial nucleic acids from the lysate obtained in step 5.

2. Method according to claim 1, wherein the mammalian cells represent human cells.

3. Method according to claim 1 or 2, wherein the samples represent body fluids, concentrates or diluted solutions thereof, or resuspended cell material collected using swabs, said cell material preferably being blood or blood concentrates.

4. Method according to any one of claims 1 to 3, wherein the microbial cells represent bacteria and/or fungal cells.

5. Method according to any one of claims 1 to 4, wherein the separation, in essence, of the liquid components from the solid components in the steps 1 and/or 3 according to claim 1 takes place with the aid of centrifugation, filtration and/or decantation of the sample.

6. Method according to any one of claims 1 to 5, wherein the selective lysis of the mammalian cells according to method step 2 of claim 1 is performed at a pH value of 8.0.

7. Method according to any one of claims 1 to 6, wherein the lysis of the microbial cells in step 5 according to claim 1 takes place at least partly mechanically, preferably by homogenising the sample using particles, preferably glass beads, particularly preferably using glass beads of size 400 to 600 µm in the presence of a buffer solution.

8. Method according to claim 7, wherein the buffer solution (buffer 2), having a pH value of 6 to 10, preferably 7.0 to 9.0, comprises
1. at least one buffer substance, preferably selected from the group containing Tris HCl, MES, MOPS, HEPES or mixtures thereof,
2. at least one complexing reagent for metal ions, in particular for divalent metal ions, preferably EDTA,
3. at least one surfactant, preferably a non-ionic surfactant, selected particularly preferably from the group containing sorbitan esters of fatty acids, polyoxyethylene sorbitan esters of fatty acids, polyoxyalkylene ethers of fatty acid alcohols, polyoxyalkylene ethers of alkyl phenols, poloxamers or mixtures thereof, and
4. optionally, a foam inhibitor, preferably selected from the group containing reagent DX and SILFOAM SRE,
and wherein the substances of the buffer solution are present during the mechanical lysis in particular in a concentration of (1) 2 to 20 mM of buffer substance, (2) 0.1 to 5 mM of complexing reagent, (3) 0.1 to 5 % (w/v) of surfactant and (4) optionally 0.1 to 1 % (v/v) of foam inhibitor at a pH value of 8.0.

9. Method according to any one of claims 1 to 8, wherein the lysis of the microbial cells in step 5 according to claim 1 takes place at least partly by adding to the suspension from step 4, according to claim 1 or to the lysate resulting from claim 7 or 8, a proteinase, preferably proteinase K, and a buffer solution (buffer 3), consisting of
1. at least one chaotropic substance selected from the group containing guanidine thiocyanate (GTC), guanidine hydrochloride (GuHCl), guanidine isothiocyanate (GIT), alkali perchlorates, alkali iodides, urea or mixtures thereof,
2. at least one surfactant, preferably a non-ionic surfactant, selected particularly preferably from the group containing sorbitan esters of fatty acids, polyoxyethylene sorbitan esters of fatty acids, polyoxyalkylene ethers of fatty acid alcohols, polyoxyalkylene ethers of alkyl phenols, poloxamers or mixtures thereof, and
3. at least one buffer substance, preferably selected from the group containing Tris, MES, MOPS, HEPES or mixtures thereof,
4. and, optionally, the liquid component from step 1 according to claim 1 and wherein the substances of the buffer solution are present during the lysis are present in particular in a concentration of (1) 1 to 5 M of chaotrope, (2) 5 to 20 % (w/v) of surfactant and (3) 20 to 100 mM of buffer substance, in particular at a pH value of 8.0,
and incubation of the mixture obtained.

10. Method according to any one of claims 1 to 9, additionally including the enrichment and/or isolation of viral nucleic acids from samples that contain, besides a mixture of microbial cells, mammalian cells and freely circulating nucleic acids, in particular extracellular microbial nucleic acids, in a liquid, viruses, wherein the viruses are separated from the solid sample components in step 1 according to claim 1 together with the liquid component of the sample.

11. Method according to one of claims 1 to 10, wherein the liquid component separated in step 1 according to claim 1, containing the extracellular, preferably microbial nucleic acids and possibly the viral nucleic acids, is combined with the lysate, obtained in step 5 according to claim 1 and containing the intracellular microbial nucleic acids, before the purification and/or concentration of the nucleic acids according to step 6 of claim 1.

12. Method according to one of the claims 1 to 10, wherein the liquid component obtained in step 1 according to claim 1, containing the extracellular microbial and possibly the viral nucleic acids and the lysate obtained in step 5 according to claim 1 and containing the intracellular microbial nucleic acids are purified and/or concentrated separately from each other.

13. Method according to any one of the claims 1 to 12, wherein the purification and concentration of the microbial and possibly the viral nucleic acids according to step 6 of claim 1 comprises the following steps:
6a) Mixing the lysate from step 5 according to claim 1 and possibly the liquid component from step 1 with a solution (binding buffer), preferably containing a mixture of (1) a linear or branched C₁-C₄ alcohol, preferably isopropanol, and (2) a buffer 3 according to claim 9, containing (1) 3 to 6 M of chaotrope, (2) 10 to 30 % (w/v) of surfactant and (3) 50 to 150 mM of buffer substance, in particular at a pH value of 8.0, preferably in a proportion of 30 - 80 % of linear or branched C₁-C₄ alcohol to 20 - 70 % of buffer 3, followed by incubation of the resulting mixture,
6b) Applying the sample to a suitable chromatographic device, preferably a chromatographic device for selectively binding the microbial and possible viral nucleic acids by adsorption, particularly preferably based on silica,
6c) optionally washing, one or more times, the bound microbial and possible viral nucleic acids,
6d) optionally eluting the microbial and possible viral nucleic acids.

14. Kit for the selective enrichment and/or isolation of intracellular microbial as well as freely circulating nucleic acids, in particular extracellular microbial nucleic acids, and possibly additional viral nucleic acids from samples that contain a mixture of microbial cells, mammalian cells and freely circulating nucleic acids, in particular extracellular microbial nucleic acids, as well as possibly additional viruses in a liquid, comprising:
1. a first buffer according to claim 1, containing (1) 0.2 to 4 M chaotrope, selected from the group containing guanidine thiocyanate (GTC), guanidine hydrochloride (GuHCl), guanidine isothiocyanate (GIT), alkali perchlorates, alkali iodides, urea or mixtures thereof, (2) 0.5 to 10 % (w/v) of non-ionic surfactant, selected from the group containing sorbitan esters of fatty acids, polyoxyethylene sorbitan esters of fatty acids, polyoxyalkylene ethers of fatty acid alcohols, polyoxyalkylene ethers of alkyl phenols, poloxamers or mixtures thereof, and (3) 2 to 100 mM of buffer substance, preferably with a pH value of 8.0,
2. at least one second buffer according to claim 8, containing (1) 5 to 50 mM of buffer substance, (2) 0.5 to 10 mM of complexing reagent, (3) 0.2 to 10 % (w/v) of surfactant and (4) optionally 0.1 to 3 % (v/v) of foam inhibitor at a pH value of 8.0, and/or, according to claim 9, containing (1) 3 to 10 M of chaotrope, (2) 10 to 40 % (w/v) of surfactant, and (3) 40 to 200 mM of buffer substance, in particular at a pH value of 8.0,
3. glass beads, preferably with a diameter of 400 to 600 µm, particularly preferably a diameter of 500 µm.

15. Kit according to claim 14, further comprising a chromatographic device for purifying and/or concentrating the microbial and possibly viral nucleic acids, preferably a chromatographic device for selectively binding the microbial and viral nucleic acids by adsorption, based particularly preferably on silica, as well as, furthermore, a binding buffer, preferably a binding buffer according to claim 13, optionally one or more washing buffers, optionally an elution buffer, as well as optionally other components to verify the enriched and/or isolated nucleic acids.

## Revendications

1. Procédé d'enrichissement sélectif et/ou d'isolement d'acides nucléiques microbiens issus d'échantillons, lesquels contiennent un mélange de cellules microbiennes, de cellules de mammifères et d'acides nucléiques circulant librement, en particulier d'acides nucléiques microbiens extracellulaires, dans un liquide, comprenant les étapes suivantes :
- 1. Séparation essentiellement de la partie liquide de l'échantillon, y compris des substances dissoutes dans ladite partie liquide telles que les acides nucléiques circulant librement, en particulier les acides nucléiques microbiens extracellulaires, de la partie solide de l'échantillon, en particulier de la partie cellulaire,
- 2. Lyse sélective des cellules de mammifères contenues dans la partie solide de l'échantillon sans lyse simultanée des cellules microbiennes, la lyse sélective des cellules de mammifères étant réalisée dans un mélange (tampon 1), comprenant
--1. au moins une substance chaotrope, choisie parmi le groupe contenant du thiocyanate de guanidinium (GTC), de l'hydrochlorure de guanidium (GuHCl), de l'isothiocyanate de guanidinium (GIT), du perchlorate alcalin, de l'iodure alcalin, de l'urée ou des mélanges de ceux-ci,
--2. au moins un tensioactif non ionique, choisi parmi le groupe contenant des esters de sorbitane d'acides gras, des esters de sorbitane polyoxyéthylène d'acides gras, de l'éther de polyoxyalkylène des alcools d'acides gras, de l'éther de polyoxyalkylène d'alkylphénols, du poloxamère ou des mélanges de ceux-ci et
--3. au moins une substance tampon, de préférence choisie parmi le groupe contenant Tris, MES, MOPS, HEPES ou des mélanges de ceux-ci,
de telle manière que la concentration desdites substances de l'échantillon comporte, après ajout de la solution tampon, (1) de 0,1 à 2 M de chaotrope, (2) de 0,5 à 5 % (p/v) de tensioactif non ionique et (3) de 2 à 50 mM de substance tampon.
- 3. Séparation essentiellement de la partie solide, y compris des cellules microbiennes, de la partie liquide, y compris des substances dissoutes dans la partie liquide, du lysat obtenu à l'étape 2,
- 4. Resuspension de la partie solide obtenue à l'étape 3,
- 5. Lyse des cellules microbiennes, lesquelles sont présentes dans la suspension obtenue à l'étape 4,
- 6. Purification et/ou concentration des acides nucléiques circulant librement, en particulier des acides nucléiques microbiens extracellulaires de la partie liquide obtenue à l'étape 1 et/ou des acides nucléiques microbiens intracellulaires du lysat obtenu à l'étape 5.

2. Procédé selon la revendication 1, les cellules de mammifères représentant des cellules humaines.

3. Procédé selon la revendication 1 ou 2, les échantillons représentant des liquides biologiques, des concentrés ou des solutions diluées du matériel cellulaire identique ou remis en suspension, lequel est prélevé à l'éponge, de préférence du sang ou des concentrés sanguins.

4. Procédé selon l'une quelconque des revendications 1 à 3, les cellules microbiennes représentant des cellules bactériennes et/ou des cellules fongiques.

5. Procédé selon l'une quelconque des revendications 1 à 4, la séparation essentiellement de la partie liquide de la partie solide s'effectuant aux étapes de procédé 1 et/ou 3 selon la revendication 1 à l'aide de la centrifugation, la filtration et/ou la décantation de l'échantillon.

6. Procédé selon l'une quelconque des revendications 1 à 5, la lyse sélective des cellules de mammifères selon l'étape de procédé 2 de la revendication 1 étant réalisée pour un pH de 8,0.

7. Procédé selon l'une quelconque des revendications 1 à 6, la lyse des cellules microbiennes de l'étape de procédé 5 selon la revendication 1 étant effectuée au moins partiellement de manière mécanique, de préférence par homogénéisation mécanique de l'échantillon moyennant l'utilisation de particules, de préférence des perles de verre, de manière particulièrement préférée moyennant l'utilisation de perles de verre dont la taille va de 400 à 600 µm en présence d'une solution tampon.

8. Procédé selon la revendication 7, la solution tampon (tampon 2) comprenant
- 1. au moins une substance tampon, de préférence choisie parmi le groupe contenant Tris-HCl, MES, MOPS, HEPES ou des mélanges de ceux-ci,
- 2. au moins un réactif de complexation pour les ions métalliques, en particulier pour les ions métalliques bivalents, de préférence l'EDTA,
- 3. au moins un tensioactif, de préférence un tensioactif non ionique, de manière particulièrement préférée choisi parmi le groupe contenant des esters de sorbitane d'acides gras, des esters de sorbitane polyoxyéthylène d'acides gras, de l'éther de polyoxyalkylène des alcools d'acides gras, de l'éther de polyoxyalkylène d'alkylphénols, du poloxamère ou des mélanges de ceux-ci et
- 4. de manière facultative, un inhibiteur de mousse, de préférence choisi parmi le groupe contenant le réactif DX et SILFOAM SRE,
et présentant un pH allant de 6 à 10, de préférence de 7,0 à 9,0,
et les substances de la solution tampon présentant pendant la lyse mécanique, en particulier selon une concentration de (1) 2 à 20 mM pour la substance tampon, (2) de 0,1 à 5 mM pour le réactif de complexation, (3) de 0,1 à 5 % (p/v) pour le tensioactif et (4) facultativement de 0,1 à 1 % (v/v) pour l'inhibiteur de mousse pour un pH de 8,0.

9. Procédé selon l'une quelconque des revendications 1 à 8, la lyse des cellules microbiennes à l'étape de procédé 5 selon la revendication 1 s'effectuant au moins partiellement par le mélange de la suspension de l'étape de procédé 4 selon la revendication 1 ou du lysat résultant de la revendication 7 ou 8 avec une protéinase, de préférence la protéinase K, et avec une solution tampon (tampon 3), comprenant
- 1. au moins une substance chaotrope, de préférence choisie parmi le groupe, contenant du thiocyanate de guanidinium (TCG), de l'hydrochlorure de guanidium (GuHCl), de l'isothiocyanate de guanidinium (GIT), du perchlorate alcalin, de l'iodure alcalin, de l'urée ou des mélanges de ceux-ci,
- 2. au moins un tensioactif, de préférence un tensioactif non ionique, choisi de manière particulièrement préférée parmi le groupe contenant des esters de sorbitane d'acides gras, des esters de sorbitane polyoxyéthylène d'acides gras, de l'éther de polyoxyalkylène des alcools d'acides gras, de l'éther de polyoxyalkylène d'alkylphénols, du poloxamère ou des mélanges de ceux-ci
- 3. au moins une substance tampon, de préférence choisie parmi le groupe contenant Tris, MES, MOPS, HEPES ou des mélanges de ceux-ci,
- 4. et le cas échéant, la partie liquide de l'étape de procédé 1 selon la revendication 1, et les substances de la solution tampon présentant pendant la lyse en particulier selon une concentration de (1) 1 à 5 m pour le chaotrope, (2) de 5 à 20 % (p/v) pour le tensioactif et (3) de 20 à 100 mM pour la substance tampon, en particulier pour un pH de 8,0 et l'incubation du mélange obtenu.

10. Procédé selon l'une quelconque des revendications 1 à 9, comprenant en outre l'enrichissement et/ou l'isolement d'acides nucléiques viraux issus d'échantillons, lesquels outre un mélange de cellules microbiennes, de cellules de mammifères et d'acides nucléiques circulant librement, en particulier d'acides nucléiques microbiens extracellulaires, dans un liquide contiennent en plus des virus, les virus de l'étape de procédé 1 selon la revendication 1 étant séparés conjointement avec la partie liquide de l'échantillon de la partie solide de l'échantillon.

11. Procédé selon l'une des revendications 1 à 10, la partie liquide étant séparée à l'étape de procédé 1 selon la revendication 1 contenant les acides nucléiques extracellulaires, de préférence microbiens, et le cas échéant les acides nucléiques viraux, avec le lysat obtenu à l'étape de procédé 5 selon la revendication 1, contenant les acides nucléiques microbiens intracellulaires ont été réunis avant la purification et/ou la concentration des acides nucléiques selon l'étape de procédé 6 de la revendication 1.

12. Procédé selon l'une des revendications 1 à 10, la partie liquide obtenue à l'étape de procédé 1 selon la revendication 1, contenant les acides nucléiques microbiens extracellulaires et le cas échéant les acides nucléiques viraux et le lysat obtenu à l'étape de procédé 5 selon la revendication 1, contenant les acides nucléiques microbiens intracellulaires, ont été purifiés et/ou concentrés en étant séparés les uns des autres.

13. Procédé selon l'une quelconque des revendications 1 à 12, la purification et la concentration des acides nucléiques microbiens et le cas échéant viraux selon l'étape 6 de la revendication 1 comprennent les étapes suivantes :
- 6a) le mélange du lysat de l'étape de procédé 5 selon la revendication 1 et le cas échéant de la partie liquide de l'étape 1 avec une solution (tampon de liaison), comprenant de préférence un mélange (1) d'un alcool linéaire ou ramifié en C₁ à C₄, de préférence l'isopropanol, et (2) d'un tampon 3 selon la revendication 9, contenant (1) de 3 à 6 M de chaotrope, (2) de 10 à 30 % (p/v) de tensioactif et (3) de 50 à 150 mM de substance tampon, en particulier pour un pH de 8,0, de préférence selon un rapport de 30 à 80 % d'un alcool linéaire ou ramifié en C₁ à C₄ sur 20 à 70 % de tampon 3 et l'incubation du mélange qui en résulte,
- 6b) le placement de l'échantillon sur un dispositif de chromatographie approprié, de préférence un dispositif de chromatographie destiné à la liaison sélective des acides nucléiques microbiens et le cas échéant viraux par adsorption, de manière particulièrement préférée sur une base de silice,
- 6c) de manière facultative le lavage une ou plusieurs fois des acides nucléiques microbiens liés et le cas échéant des acides nucléiques viraux,
- 6d) de manière facultative l'élution des acides nucléiques microbiens et le cas échéant des acides nucléiques viraux.

14. Kit destiné à l'enrichissement sélectif et/ou l'isolement d'acides nucléiques microbiens intracellulaires, ainsi que d'acides nucléiques circulant librement, en particulier d'acides nucléiques microbiens extracellulaires et le cas échéant l'ajout d'acides nucléiques viraux issus d'échantillons, lesquels contiennent un mélange de cellules microbiennes, de cellules de mammifères et d'acides nucléiques circulant librement, en particulier d'acides nucléiques microbiens extracellulaires, ainsi que le cas échéant l'ajout de virus dans un liquide, comprenant :
- 1. un premier tampon selon la revendication 1 contenant (1) de 0,2 à 4 M de chaotrope, choisi parmi le groupe contenant du thiocyanate de guanidinium (GTC), de l'hydrochlorure de guanidium (GuHCl), de l'isothiocyanate de guanidinium (GIT), du perchlorate alcalin, de l'iodure alcalin, de l'urée ou des mélanges de ceux-ci, (2) de 0,5 à 10 % (p/v) de tensioactif non ionique, choisi parmi le groupe contenant des esters de sorbitane d'acides gras, des esters de sorbitane polyoxyéthylène d'acides gras, de l'éther de polyoxyalkylène des alcools d'acides gras, de l'éther de polyoxyalkylène d'alkylphénols, du poloxamère ou des mélanges de ceux-ci et (3) de 2 à 100 mM de substance tampon, de préférence avec un pH de 8,0,
- 2. au moins un deuxième tampon selon la revendication 8 contenant (1) de 5 à 50 mM de substance tampon, (2) de 0,5 à 10 mM de réactif de complexation, (3) de 0,2 à 10 % (p/v) de tensioactif et (4) de manière facultative de 0,1 à 3 % (v/v) d'inhibiteur de mousse pour un pH de 8,0, et/ou selon la revendication 9 contenant (1) de 3 à 10 M de chaotrope, (2) de 10 à 40 % (p/v) de tensioactif et (3) de 40 à 200 mM de substance tampon, en particulier pour un pH de 8,0,
- 3. des perles de verre, de préférence dont le diamètre va de 400 à 600 µm, de manière particulièrement préférée de 500 µm de diamètre.

15. Kit selon la revendication 14, comprenant en outre un dispositif de chromatographie pour la purification et/ou la concentration d'acides nucléiques microbiens et le cas échéant d'acides nucléiques viraux, de préférence un dispositif de chromatographie destiné à la liaison sélective des acides nucléiques microbiens et viraux par adsorption, de manière particulièrement préférée sur base de silice, ainsi qu'en outre un tampon de liaison, de préférence un tampon de liaison selon la revendication 13, facultativement un ou plusieurs tampons de lavage, facultativement un tampon d'élution, ainsi que facultativement d'autres composants pour détecter la présence d'acides nucléiques enrichis et/ou isolés.
